# EUROPEAN PATENT SPECIFICATION

(11) **EP 0 979 272 B1**
(45) Date of publication and mention of the grant of the patent: **24.09.2008**
(21) Application number: 98913379.8
(22) Date of filing: 31.03.1998
(51) Int. Cl.: C12N 15/00, C12N 15/12, C12N 5/10, C07K 14/72, C07K 16/18, C12Q 1/68, A01K 67/027, A61K 39/395, G01N 33/50, G01N 33/569

(54) **STRL33, A HUMAN FUSION ACCESSORY FACTOR ASSOCIATED WITH HIV INFECTION**
MENSCHLICHER FUSIONS-ZUSATZFAKTOR, STRL33, DER MIT HIV-INFEKTION VERBUNDEN IST
FACTEUR HUMAIN DE FUSION ACCESSOIRE STRL33 ASSOCIE A L'INFECTION A VIH

(30) Priority: 31.03.1997 US 42880 P
(43) Date of publication of application: 16.02.2000
(73) Proprietor: THE GOVERNMENT OF THE UNITED STATES OF AMERICA, as represented by THE SECRETARY, DEPARTMENT OF HEALTH AND HUMAN SERVICES, Rockville, MD 20852 (US)
(72) Inventor: FARBER, Joshua, M., Washington, DC 20008 (US); LIAO, Fang, Rockville, MD 20852 (US); ALKHATIB, Ghalib, Bethesda, MD 20892 (US); BERGER, Edward, A., Rockville, MD 20852 (US)
(74) Representative: Wuesthoff, Franz
(86) International application number: PCT/US1998/006517
(87) International publication number: WO 1998/044098

(56) References cited:
- EP-A- 0 834 563
- FENG Y ET AL: "HIV-1 ENTRY COFACTOR: FUNCTIONAL CDNA CLONING OF A SEVEN- TRANSMEMBRANE, G PROTEIN-COUPLED RECEPTOR" SCIENCE, vol. 272, 10 May 1996, pages 872-877, XP002027999
- ALKHATIB G ET AL: "CC CKR5: A RANTES, MIP-1ALPHA, MIP-1BETA RECEPTOR AS A FUSION COFACTOR FOR MACROPHAGE-TROPIC HIV.1" SCIENCE, vol. 272, 28 June 1996, pages 1955-1958, XP002055728
- DORANZ B J ET AL: "A DUAL-TROPIC PRIMARY HIV-1 ISOLATE THAT USED FUSIN AND THE BETA- CHEMOKINE RECEPTORS CKR-5, CKR-3, AND CKR-2B AS FUSION COFACTORS" CELL, vol. 85, no. 7, 28 June 1996, pages 1149-1158, XP002028000
- M. MOSER: "Chemokines and HIV: a remarkable synergism" TRENDS IN MICROBIOLOGY, vol. 5, no. 3, March 1997, pages 88-90, XP002077875 elsevier science ltd., Amsterdam, NL
- F. LIAO ET AL.: "STRL33, a novel chemokine receptor-like protein, functions as a fusion cofactor for both macrophage-tropic and T-cell line-tropic HIV-1" J. EXP. MED., vol. 185, no. 11, 2 June 1997, pages 2015-2023, XP002077717 ROCKEFELLER UNIV. PRESS, US
- H.K. DENG ET AL.: "Expression cloning of new receptors used by simian and human immunodeficiency viruses" NATURE, (1997 JUL 17) 388 (6639) 296-300. JOURNAL CODE: NSC. ISSN: 0028-0836., XP002077715
- G. ALKHATIB ET AL.: "A new SIV co-receptor, STRL33" NATURE, (17 JUL 1997) VOL. 388, NO. 6639, PP. 238-238. PUBLISHER: MACMILLAN MAGAZINES LTD, PORTERS SOUTH, 4 CRINAN ST, LONDON, ENGLAND N1 9XW. ISSN: 0028-0836., XP002077716
- M. LOETSCHER ET AL.: "TYMSTR, a putative chemokine receptor selectively expressed in activated t cells, exhibits HIV-1 coreceptor function" CURRENT BIOLOGY, vol. 7, no. 9, 1 September 1997, pages 652-660, XP002077876 LONDON, UK

## Description

### 1. FIELD OF THE INVENTION

The present invention pertains generally to *in vitro* and *in vivo* models for the study of human immunodeficiency virus (HIV) infection and the effectiveness of anti-HIV therapeutics and specifically to a polypeptide, designated STRL33, which plays a role in the membrane fusion step of HIV infection.

### 2. BACKGROUND OF THE INVENTION

The HIV infection cycle begins with the entry of the virus into the target cell. The human CD4 molecule is the primary receptor recognized by HIV. The binding of the HIV envelope glycoprotein (*env*) to the CD4 receptor results in the fusion of virus and cell membranes, which in turn facilitates virus entry into the host. The eventual expression of *env* on the surface of the HIV-infected host cell enables this cell to fuse with uninfected, CD4-positive cells, thereby spreading the virus.

Recent studies have shown that this HIV fusion process occurs with a wide range of human cell types that either express human CD4 endogenously or have been engineered to express human CD4. The fusion process, however, does not occur with nonhuman cell types engineered to express human CD4. Although such nonhuman cells can still bind *env,* membrane fusion does not follow. The disparity between human and nonhuman cell types exists apparently because membrane fusion requires the coexpression of human CD4 and an accessory factor specific to human cell types. Because they lack this accessory factor, nonhuman cell types engineered to express only human CD4 are incapable of membrane fusion, and are thus nonpermissive for HIV infection. To date there has been no report of any stable, nonhuman cell line that is permissive for HIV infection as a result of human CD4 and STRL33 coexpression.

The importance of human CD4 and STRL33 coexpression also impacts the establishment of a successful small animal model. The development of a small animal model is crucial to the study of HIV infection and the effectiveness of anti-HIV therapeutics. In recent years, researchers have bred transgenic animals having cells that express human CD4. See, for example, Dunn et al., Human immunodeficiency virus type 1 infection of human CD4-transgenic rabbits, J. Gen. Vir. 76:1327-1336 (1995); Snyder et al., Development and Tissue-Specific Expression of Human CD4 in Transgenic Rabbits, Mol. Reprod. & Devel. 40:419-428 (1995); Killeen et al., Regulated Expression of Human CD4 Rescues Helper T-Cell Development in Mice Lacking Expression of Endogenous CD4, EMBO J. 12:1547-1553 (1993); Forte et al., Human CD4 Produced in Lymphoid Cells of Transgenic Mice Binds HIV p120 and Modifies the Subsets of Mouse T-Cell Populations, Immunogenetics 38:455-459 (1993). These animals, however, have low susceptibility to HIV infection, presumably because of the lack expression of a co-receptor for. To date, there has been no report of any transgenic animal that is significantly susceptible to HIV infection as a result of human CD4 and STRL33 coexpression.

B. Moser describes in TRENDS IN MICROBIOLOGY, vol. 5, no. 3, pp. 88-90, a synergism between chemokines and HIV. Y. Feng et al. discloses in SCIENCE, vol. 272, 28 June 1996, pp. 872-877 a cofactor for HIV-1 fusion and entry. EP 0 834 563, published on 1998-04-08 and claiming a priority date of 1996-09-25, discloses a human 7-transmembrane receptor, member of the chemokine receptor family, named STRL-33.

Without an effective vaccine, the number of individuals infected with HIV will likely increase substantially. Furthermore, in the absence of effective therapy, most individuals infected with HIV will develop acquired immune deficiency syndrome (AIDS) and succumb to either opportunistic infections and malignancies that result from the deterioration of the immune system, or the direct pathogenic effects of the virus. Despite the present availability of some anti-HIV agents that slow disease progression, a pressing need remains for more effective therapeutics and drug combinations.

It is apparent from the foregoing that a need exists for *in vitro* and *in vivo* models suitable to the study of HIV infection and the effectiveness of anti-HIV therapeutics. By the same token, the need remains for more effective anti-HIV therapeutics.

### SUMMARY OF THE INVENTION

The susceptibility to HIV infection depends on the cell surface expression of the human CD4 molecule and a heretofore unidentified human fusion accessory factor. The present invention relates to the use of a fusion accessory factor, designated STRL33. A comparison of the nucleotide sequence of the cDNA encoding STRL33 against a computer database revealed that STRL33 is a member of the 7-transmembrane segment superfamily of G-protein-coupled cell surface molecules (GPCR). Many of the superfamily members function as ligand receptors in relation, for example, to peptide hormones, neurotransmitters, and chemokines. STRL33 has no known ligand.

The identification of STRL33 adds to the recent discoveries on the roles of chemokine receptors in the pathobiology of HIV-1 infection. STRL33 is a GPCR that can function with CD4 to mediate fusion with cells bearing HIV-1 Envs from both laboratory-adapted TCL-tropic variants and from M-tropic variants. In this regard, STRL33 can mediate fusion with a wider range of Envs than can the major cofactors fusin/CXCR4 and CCR5. While the role of STRL33 in the biology of viral infection is unknown, the present invention demonstrates both that STRL33 is functional as a cofactor for HIV-1 Env-mediated fusion and that the STRL33 gene is expressed in cells and tissues that are natural targets for HIV-1.

A key aspect of the present invention is the discovery that STRL33 plays a role in the membrane fusion step of HIV infection. The establishment of stable, nonhuman cell lines and transgenic mammals having cells that coexpress human CD4 and STRL33 provides valuable tools for the continuing research of HIV infection and the development of more effective anti-HIV therapeutics. In addition, antibodies which bind STRL33, isolated and purified peptide fragments of STRL33, and STRL33-binding agents, capable of blocking membrane fusion between HIV and target cells represent potential anti-HIV therapeutics.

In particular, the present invention provides a recombinant host cell stably transformed with a polynucleotide encoding STRL33 polypeptide as defined in claim 1 and a recombinant host cell stable transformed with a polynucleotide encoding STRL33 polypeptide and a polynucleotide encoding CD4 polypeptide as defined in claim 2.

Further, the present invention relates to the use of an anti-STRL33 specific antibody or epitope binding fragment thereof in the production of a medicament for inhibiting membrane fusion between HIV and a STRL33 positive target cell or between an HIV-infected cell and a CD4 positive STRL33 positive uninfected cell as set forth in claim 5, and to the use of an anti-STRL33 specific antibody or epitope binding fragment thereof, or a STRL33 specific antisense nucleic acid in the production of a medicament for treating a subject having or at risk of having an HIV infection or HIV-related disorder as defined in claim 24.

Yet further, the present invention provides a transgenic non-human animal expressing STRL33 and CD4 as set forth in claim 14, and a transgenic non-human animal having a transgene disrupting or interfering with expression of STRL33 as set forth in claim 21.

Still further, the present invention provides a method for identifying a composition which blocks membrane fusion between HIV and a STRL33 positive target cell or between an HIV-infected cell and a STRL33 positive uninfected cell according to claim 10, and a method for producing a transgenic non-human animal expressing STRL33 and CD4, which naturally do not occur in said non-human animal, according to claim 17, and in vitro methods for detecting the susceptibility of a cell to HIV infection according to claims 32 and 37.

### BRIEF DESCRIPTION OF THE FIGURES

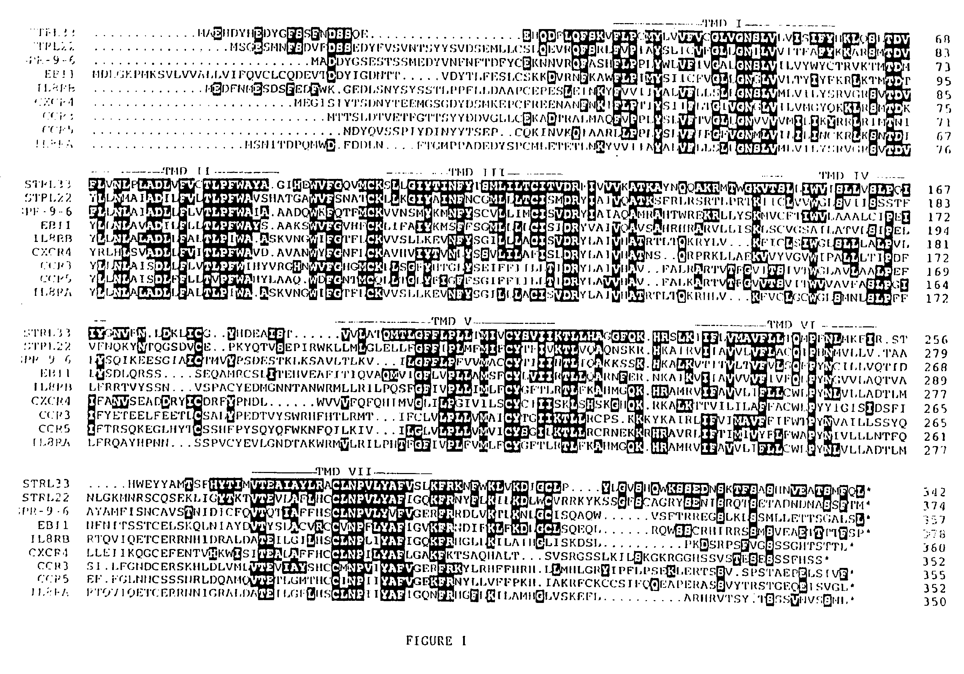

Figure 1 shows the alignment of the STRL33 predicted amino acid sequence with the selected GPCRs STRL22, GPR-9-6, EBI1, IL8RB, CXCR4, CCR3, CCR5 and IL8RA. Numbers at the right indicate the positions of the residues at the end of each line of sequence. Solid backgrounds highlight matches between STRL33 and the other receptors. Dots indicate gaps introduced for optimal alignment. Putative TMDs I-VII are indicated by bars. The alignments were generated using the PileUp program of the Wisconsin Sequence Analysis Package of Genetics Computer Group, Madison, WI.

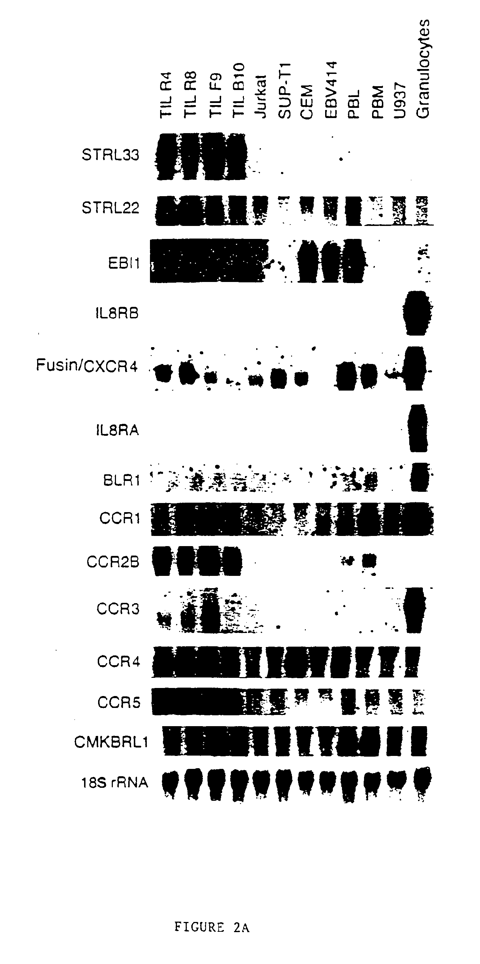

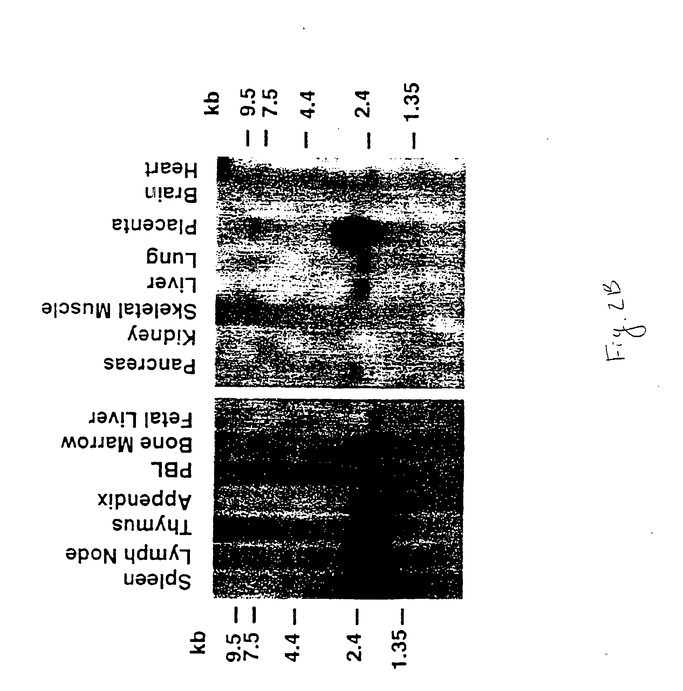

Figure 2 shows the expression STRL33 and other GPCR genes. Figure 2A. The expression of STRL33, genes for known chemokine receptors, and genes for selected orphan GPCRs in leukocytes. Fifteen micrograms of total RNA were electrophoresed on 1.2% agarose-formaldehyde gels, transferred to nitrocellulose membranes and hybridized with the probes indicated on the left. A total of six membranes were used for hybridizations, and adequate removal of signal was documented before repeat probings. Film exposure times ranged from overnight for the IL8RA and IL8RB blots to 13 d for the CXCR4 blot. Probings were done using an oligonucleotide complementary to 18S RNA in order to demonstrate amounts of RNA loaded per lane and a representative blot is shown. Figure 2B. The expression of STRL33 in human tissues. Blots were prepared by the supplier (Clontech) from 1.2% agarose-formaldehyde gels containing approximately 2 µg poly (A)⁺ RNA per lane. Hybridizations were done using a ³²P-labelled STRL33 ORF probe and blots were washed according to the supplier's instructions. Membranes were exposed to film using an intensifying screen. The blot prepared from lymphoid tissue (left) was exposed for 2 d, and the blot from other selected tissues (right) was exposed for 8 d.

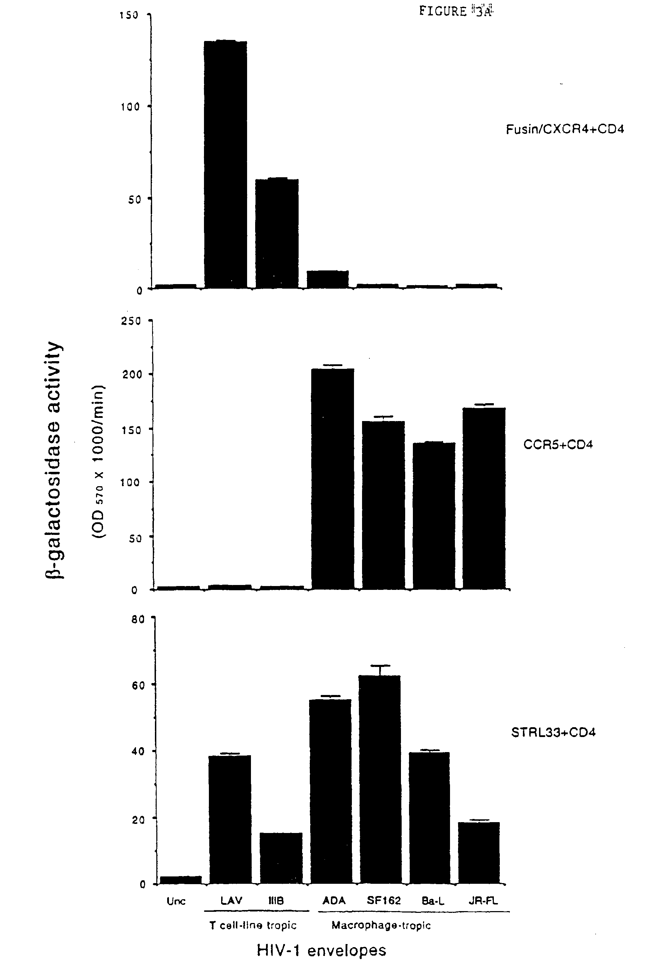

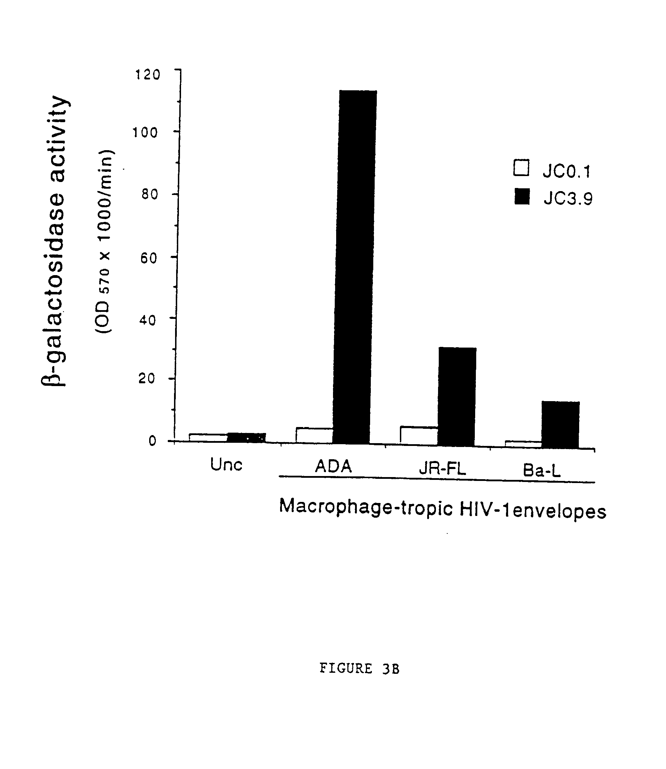

Figure 3 shows the activity of STRL33 as a fusion cofactor. Figure 3A. NIH 3T3 cells were transfected with DNAs encoding fusin/CXCR4 or CCR5 or STRL33, and infected with vaccinia recombinants encoding CD4 and T7 RNA polymerase. HeLa cells were infected with a vaccinia recombinant containing *LacZ* under control of a T7 promoter and infected separately with vaccinia recombinants encoding the indicated Envs. Unc is a mutant Env that cannot be cleaved to gp120 and gp41 and cannot mediate fusion. Cell fusion was quantified by measuring β-Gal activity. NIH 3T3 cells transfected with the STRL33 cDNA but not infected with virus vCB-3 encoding CD4 did not fuse with cells expressing any of the Envs. Results of one experiment are shown. STRL33 also mediated fusion with cells expressing both TCL-tropic and M-tropic Envs in four additional experiments. Figure 3B. Jurkat cell line JCO.1, transfected with vector control, and Jurkat cell line JC3.9, transfected with the STRL33.1 cDNA, were infected with vaccinia recombinants encoding the T7 RNA polymerase and CD4. Jurkat cells were incubated with recombinant vaccinia-infected HeLa cells and fusion was measured as in A.

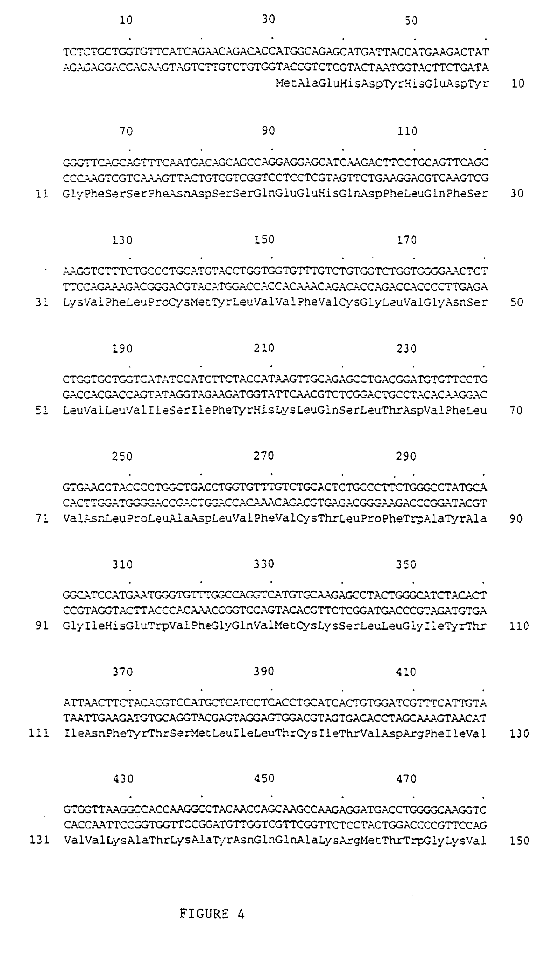

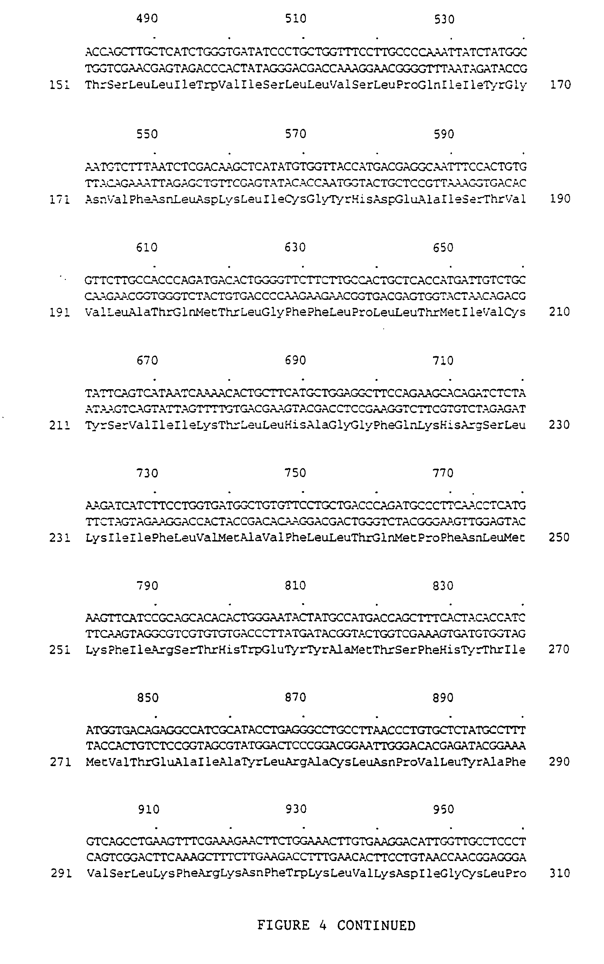

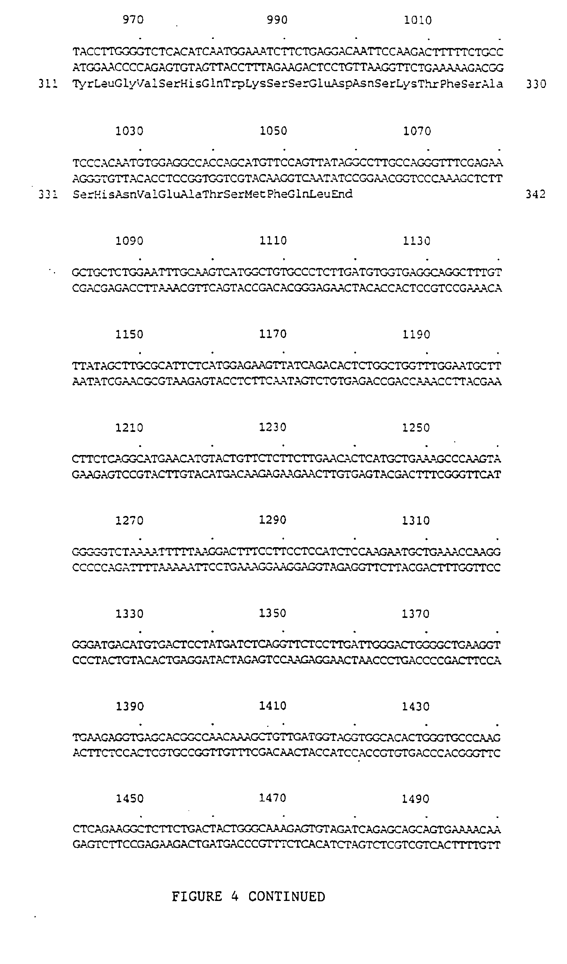

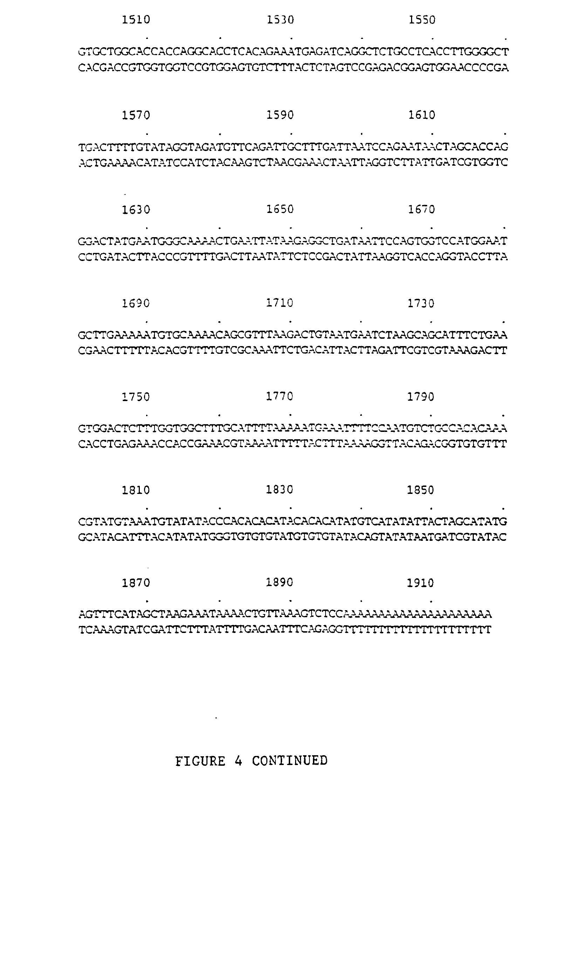

Figure 4 shows the nucleotide and deduced amino acids sequence of STRL33.

### DETAILED DESCRIPTION OF PREFERRED EMBODIMENTS

In accordance with the present invention, the STRL33 refers to a cellular protein of the 7-transmembrane segment superfamily of G-protein-coupled cell surface molecules that is associated with the fusion of virus and target cell membranes in HIV infection. The essential role of STRL33 in the membrane fusion step of HIV infection was determined by functional assay of the effects of recombinant STRL33 (*i*.*e.,* assay by vaccinia cell fusion system or HIV infection).

### cDNA ENCODING STRL33 AND STRL33 POLYPEPTIDE

To identify chemokine receptors expressed in T cells, we used RT-PCR with poly(A)+ RNA prepared from tumor infiltrating lymphocytes (TIL line F9) and pools of degenerate primers based on conserved sequences in the transmembrane domains (TMDs) of known chemokine receptors. A sequence encoding a GPCR, designated STRL33, for seven transmembrane domain receptor from lymphocytes, clone 33, is isolated. Southern blot analysis of human genomic DNA digested with BamH I, Hind III and Pst I revealed a single STRL33 gene, and using STRL33-specific primers and DNA prepared from a panel of human-hamster hybrid cell lines, STRL33 was localized to chromosome 3. This raises the possibility that STRL33 is in the cluster of genes for chemokine receptors CCR1, 2, 3, and 5 at 3p21, although the CCR proteins show > 50% amino acid identity among themselves, demonstrating significantly closer relationships than what is seen in comparisons with STRL33 (see below).

Screening of a non-amplified lambda cDNA library prepared from F9 TIL revealed an abundance for STRL33 mRNA of approximately 0.01%. Ten cDNA clones were isolated and by restriction enzyme digestion, 3 size classes were identified. Representative cDNAs from each class, STRL33.1, 33.2, and 33.3, respectively, were evaluated in more detail by restriction analysis and partial sequencing, revealing that size differences among the clones were due to 5' non-translated regions that differed not only in length but in their sequences. The complete sequence of STRL33.1, the sequence of the 5' non-translated region of STRL33.2, and the sequence of the 5' non-translated region and open reading frame (ORF) of 33.3 have been submitted to GenBank with accession numbers U73529, U73530, and U73531 respectively. STRL33.1 contained 1897 nucleotides, excluding the poly(A) tail, with an ORF encoding a predicted protein of 342 amino acids (Fig.1). The predicted initiator codon was in a favorable context for initiation (33) and could be assigned unambiguously since, with the reading frame fixed by comparison with other GPCRs, it was the first ATG following an in-frame stop codon in cDNA 33.3, and the first in-frame ATG in cDNAs 33.1 and 33.2. ORF sequence was also determined from cDNAs other than 33.1, and the sequence of the entire 33.1 ORF was confirmed in independent clones. Differences were noted, however, between the ORFs of 33.1 and 33.3 at two positions: a second position change in the 25th codon (GAC - GCC), replacing D25 with A, and a silent third position change in the 103 rd codon. The silent change in the 103 rd codon was present in a second independent clone, while the 25th codon change has not been independently verified. At least the silent change probably represents a true polymorphism in the F9 TIL. The 5' non-translated regions of 33.1, .2, and .3 were 30, 135, and 1462 nucleotides respectively, and sequence comparisons revealed that differences among these regions were due, at least in part, to alternative splicing. Not surprisingly, the long additional 5' non-translated sequence of 33.3 contained many ATG sequence (but no significant ORFs) suggesting that the 33.3 mRNA may not be translated efficiently, and although we could detect a 33.3-specific mRNA by Northern analysis (see below), the 33.3 cDNA may be derived from an incompletely processed mRNA. Extensive processing, yielding mRNAs with alternative 5' exons, is well documented among the chemoattractant receptors (2).

Comparison of STRL33 with sequences in the databases using BLAST (34) revealed no identical sequences but greatest similarity to orphan GPCRs and related chemokine receptors, and alignments between STRL33 and selected related sequences is shown in Fig. 1. Percent identities between STRL33 and orphan receptors STRL22 (22), GPR-9-6 (unpublished, GenBank accession number U45982) and EBI1 (35) are 37%, 32% and 32% respectively and between STRL33 and chemokine receptors IL8RB (CXCR2), fusin/CXCR4, CCR3, CCR5 and IL8RA (CXCR1) are 30%, 30%, 30%, 29% and 28% respectively. STRL22 is an orphan receptor that, like STRL33, was isolated from the F9 TIL (22).

Fig.1 shows that similarities among the receptors are greatest in the TMDs, as is typical of GPCRs. Like other GPCRs, STRL33 includes a site for N-linked glycosylation in the N terminal domain (N16), cysteines in extracellular loops one and two (C102 and C180), and multiple serines in the carboxy terminal domain (1). While there is no signature sequence motif for the chemokine receptors, the STRL33 sequence does contain some features characteristic of chemokine receptors, including an acidic N-terminal domain with paired acidic residues (E8 and D9, E21 and E22) (2), a short basic third intracellular loop, an alanine in place of the proline that is conserved in non-chemokine receptor GPCRs in the second intracellular loop (A134), a paired cysteine and tyrosine in TMD V (C210 and Y211) and a cysteine in TMD VII (C282). In contrast, some residues typical for chemokine receptors are absent from STRL33, including cysteine residues in the N-terminal domain and in the third extracellular loop. Multiple sequence alignment (PileUp, Genetics Computer Croup, Madison, WI) places STRL33 in a group of orphan receptors including STRL22, GPR-9-6 and EBI1 separate from the groupings of the CXCRs on one hand and the CCRs on the other.

RNA expression was analyzed by Northern blot of total RNA for STRL33 and other related receptors in leukocyte populations and lines and in human tissues. As shown in Fig. 2A, the STRL33 cDNA probe hybridized to a broad band at approximately 2 kb that was prominent in both CD4+ (R4, F9 and B10) and CD8+ (R8) TIL with low level signal in PBL but not in other cells tested, including immortalized CD4+ T cell lines . Using a probe from the 5' non-translated sequences specific for the STRL33.3 mRNA, we also detected a band at approximately 3.6 kb in the F9 and B10 TIL after long exposure, not shown in Fig.2A.

Fig. 2A shows significant differences in expression of various chemokine receptor and orphan receptor genes among the leukocytes. Of particular interest is the demonstration of heterogeneity of receptor gene expression among T cell preparations. In general, receptor gene expression is higher among the TIL than in T cell lines or PBL, although even among the TIL there are significant differences. The CD4+ F9 TIL, for example, show a significant signal for CCR3. This is noteworthy, since although CCR3 can serve in vitro as a coreceptor for HIV-1 (13), speculation on a role for CCR3 in HIV-1 infection has been constrained by the assumption that CCR3 expression is limited to eosinophils.

Fig. 2B shows the expression of the STRL33 gene in selected human tissues. There is an mRNA species of approximately 2.1 kb prominently expressed in lymphoid tissue; a prominent species of approximately 2.5 kb in placenta; low-abundance species of 2.1-2.4 kb expressed in pancreas, liver, lung and heart; and low-abundance, larger species in a variety of tissues. The conspicuous expression of the STRL33 gene in T cells and lymphoid tissues is consistent with the presumption that STRL33 is a chemokine receptor.

HEK 293 cells and Jurkat cells were transfected with expression vectors containing the STRL33.1 ORF and vector control DNA, and cell lines were derived as described in the Examples. Cell lines expressing the highest levels of STRL33 RNA were tested in a fluorometeric calcium flux assay for responses to chemokines. The STRL33-transfected HEK 293 and/or Jurkat cell lines were tested with platelet factor 4, IL8, IP-10, HuMig, SDF-1, MIP-1∝, MIP-1β, RANTES, MCP-1, MCP-2, MCP-3, MCP-4, I309 and lymphotactin and no responses were found.

The present invention describes substantially pure STRL33 polypeptide. The term "substantially pure" as used herein refers to STRL33 which is substantially free of other proteins, lipids, carbohydrates or other materials with which it is naturally associated. The substantially pure polypeptide will yield a single major band on a reducing or a nonreducing polyacrylamide gel. The purity of the STRL33 polypeptide can also be determined by amino-terminal amino acid sequence analysis. STRL33 polypeptide includes functional fragments of the polypeptide, as long as the activity of STRL33 remains.

The invention describes polynucleotides encoding the STRL33 polypeptide. These polynucleotides include DNA, cDNA and RNA sequences which encode STRL33. It is understood that all polynucleotides encoding all or a portion of STRL33 may also be used herein, as long as they encode a polypeptide with STRL33 activity. Such polynucleotides include naturally occurring, synthetic, and intentionally manipulated polynucleotides. For example, STRL33 polynucleotide may be subjected to site-directed mutagenesis. The polynucleotide sequence for STRL33 also includes antisense sequences. The polynucleotides used in the invention include sequences that are degenerate as a result of the genetic code. There are 20 natural amino acids, most of which are specified by more than one codon. Therefore, all degenerate nucleotide sequences may be used in the invention as long as the amino acid sequence of STRL33 polypeptide encoded by the nucleotide sequence is functionally unchanged.

The polynucleotide encoding STRL33 includes SEQ ID NO:1 (Figure 4) as well as nucleic acid sequences complementary to Figure 4. A complementary sequence may include an antisense nucleotide. When the sequence is RNA, the deoxynucleotides A, G, C, and T of the nucleic acid of Figure 4 are replaced by ribonucleotides A, G, C, and U, respectively. Also included are fragments of the above-described nucleic acid sequences that are at least 15 bases in length, which is sufficient to permit the fragment to selectively hybridize to DNA that encodes the protein of Figure 4 under physiological conditions. Specifically, the fragments should hybridize to DNA encoding STRL33 protein under moderately stringent conditions.

In nucleic acid hybridization reactions, the conditions used to achieve a particular level of stringency will vary, depending on the nature of the nucleic acids being hybridized. For example, the length, degree of complementarity, nucleotide sequence composition (*e*.*g.,* GC v. AT content), and nucleic acid type (*e*.*g.,* RNA v. DNA) of the hybridizing regions of the nucleic acids can be considered in selecting hybridization conditions. An additional consideration is whether one of the nucleic acids is immobilized, for example, on a filter.

An example of progressively higher stringency conditions is as follows: 2 x SSC/0.1% SDS at about room temperature (hybridization conditions); 0.2 x SSC/0.1% SDS at about room temperature (low stringency conditions); 0.2 x SSC/0.1% SDS at about 42°C (moderate stringency conditions); and 0.1 x SSC at about 68°C (high stringency conditions). Washing can be carried out using only one of these conditions, e.g., high stringency conditions, or each of the conditions can be used, *e*.*g*., for 10-15 minutes each, in the order listed above, repeating any or all of the steps listed. However, as mentioned above, optimal conditions will vary, depending on the particular hybridization reaction involved, and can be determined empirically.

Minor modifications of the STRL33 primary amino acid sequence may result in proteins which have substantially equivalent activity as compared to the STRL33 polypeptide described herein. Such proteins include those as defined by the term having essentially the amino acid sequence of the polypeptide of FIGURE 4. Such modifications may be deliberate, as by site-directed mutagenesis, or may be spontaneous. All of the polypeptides produced by these modifications are included herein as long as the biological activity of STRL33 still exists. Further, deletion of one or more amino acids can also result in a modification of the structure of the resultant molecule without significantly altering its biological activity. This can lead to the development of a smaller active molecule which would have broader utility. For example, one can remove amino or carboxy terminal amino acids which are not required for STRL33 biological activity.

The STRL33 polypeptide used in the invention encoded by the polynucleotide described in the invention includes the disclosed sequence (FIGURE 4) and conservative variations thereof. The term "conservative variation" as used herein denotes the replacement of an amino acid residue by another, biologically similar residue. Examples of conservative variations include the substitution of one hydrophobic residue such as isoleucine, valine, leucine or methionine for another, or the substitution of one polar residue for another, such as the substitution of arginine for lysine, glutamic for aspartic acid, or glutamine for asparagine, and the like. The term "conservative variation" also includes the use of a substituted amino acid in place of an unsubstituted parent amino acid provided that antibodies raised to the substituted polypeptide also immunoreact with the unsubstituted polypeptide.

DNA sequences used in the invention can be obtained by several methods. For example, the DNA can be isolated using hybridization techniques which are well known in the art. These include, but are not limited to: 1) hybridization of genomic or cDNA libraries with probes to detect homologous nucleotide sequences, 2) polymerase chain reaction (PCR) on genomic DNA or cDNA using primers capable of annealing to the DNA sequence of interest, and 3) antibody screening of expression libraries to detect cloned DNA fragments with shared structural features.

Preferably the STRL33 polynucleotide is derived from a mammalian organism, and most preferably from human. Screening procedures which rely on nucleic acid hybridization make it possible to isolate any gene sequence from any organism, provided the appropriate probe is available. Oligonucleotide probes, which correspond to a part of the sequence encoding the protein in question, can be synthesized chemically. This requires that short, oligopeptide stretches of amino acid sequence must be known. The DNA sequence encoding the protein can be deduced from the genetic code, however, the degeneracy of the code must be taken into account. It is possible to perform a mixed addition reaction when the sequence is degenerate. This includes a heterogeneous mixture of denatured double-stranded DNA. For such screening, hybridization is preferably performed on either single-stranded DNA or denatured double-stranded DNA.

Hybridization is particularly useful in the detection of cDNA clones derived from sources where an extremely low amount of mRNA sequences relating to the polypeptide of interest are present. In other words, by using stringent hybridization conditions directed to avoid non-specific binding, it is possible, for example, to allow the autoradiographic visualization of a specific cDNA clone by the hybridization of the target DNA to that single probe in the mixture which is its complete complement (Wallace, et al., Nuc/. Acid Res., 9:879, 1981; Maniatis, et al., Molecular Cloning: A Laboratory Manual, Cold Spring Harbor. N.Y. 1989).

The development of specific DNA sequences encoding STRL33 can also be obtained by: 1) isolation of double-stranded DNA sequences from the genomic DNA; 2) chemical manufacture of a DNA sequence to provide the necessary codons for the polypeptide of interest; and 3) *in vitro* synthesis of a double-stranded DNA sequence by reverse transcription of mRNA isolated from a eukaryotic donor cell. In the latter case, a double-stranded DNA complement of mRNA is eventually formed which is generally referred to as cDNA.

Of the three above-noted methods for developing specific DNA sequences for use in recombinant procedures, the isolation of genomic DNA isolates is the least common. This is especially true when it is desirable to obtain the microbial expression of mammalian polypeptides due to the presence of introns.

The synthesis of DNA sequences is frequently the method of choice when the entire sequence of amino acid residues of the desired polypeptide product is known. When the entire sequence of amino acid residues of the desired polypeptide is not known, the direct synthesis of DNA sequences is not possible and the method of choice is the synthesis of cDNA sequences. Among the standard procedures for isolating cDNA sequences of interest is the formation of plasmid- or phage-carrying cDNA libraries which are derived from reverse transcription of mRNA which is abundant in donor cells that have a high level of genetic expression. When used in combination with polymerase chain reaction technology, even rare expression products can be cloned. In those cases where significant portions of the amino acid sequence of the polypeptide are known, the production of labeled single or double-stranded DNA or RNA probe sequences duplicating a sequence putatively present in the target cDNA may be employed in DNA/DNA hybridization procedures which are carried out on cloned copies of the cDNA which have been denatured into a single-stranded form (Jay, et al., Nucl. Acid Res., 11:2325, 1983).

A cDNA expression library, such as lambda gt11, can be screened indirectly for STRL33 peptides having at least one epitope, using antibodies specific for STRL33. Such antibodies can be either polyclonally or monoclonally derived and used to detect expression product indicative of the presence of STRL33 cDNA.

DNA sequences encoding STRL33 can be expressed *in vitro* by DNA transfer into a suitable host cell. "Host cells" are cells, excluding human embryonic stem cells, in which a vector can be propagated and its DNA expressed. The term also includes any progeny of the subject host cell. It is understood that all progeny may not be identical to the parental cell since there may be mutations that occur during replication. However, such progeny are included when the term "host cell" is used. Methods of stable transfer, meaning that the foreign DNA is continuously maintained in the host, are known in the art.

The recombinant host cell according to the present invention is stably transformed with a polynucleotide encoding STRL33 polypeptide and optionally with a polynucleotide encoding CD4 polypeptide, wherein the cell co-expresses STRL33 and CD4 polypeptide, provided that the cell is not a human embryonic stem cell.

In the present invention, the STRL33 polynucleotide sequences may be inserted into a recombinant expression vector. The term "recombinant expression vector" refers to a plasmid, virus or other vehicle known in the art that has been manipulated by insertion or incorporation of the STRL33 genetic sequences. Such expression vectors contain a promoter sequence which facilitates the efficient transcription of the inserted genetic sequence of the host. The expression vector typically contains an origin of replication, a promoter, as well as specific genes which allow phenotypic selection of the transformed cells. Vectors suitable for use in the present invention include, but are not limited to the T7-based expression vector for expression in bacteria (Rosenberg, et al., Gene, 56:125, 1987), the pMSXND expression vector for expression in mammalian cells (Lee and Nathans, J. Biol. Chem., 263:3521, 1988) and baculovirus-derived vectors for expression in insect cells. The DNA segment can be present in the vector operably linked to regulatory elements, for example, a promoter (e.g., T7, metallothionein I, or polyhedrin promoters).

Polynucleotide sequences encoding STRL33 can be expressed in either prokaryotes or eukaryotes. Hosts can include microbial, yeast, insect and mammalian organisms. The host cell is not a human embryonic stem cell. Methods of expressing DNA sequences having eukaryotic or viral sequences in prokaryotes are well known in the art. Biologically functional viral and plasmid DNA vectors capable of expression and replication in a host are known in the art. Such vectors are used to incorporate DNA sequences of the invention.

Transformation of a host cell with recombinant DNA may be carried out by conventional techniques as are well known to those skilled in the art. Where the host is prokaryotic, such as *E*. *coli*, competent cells which are capable of DNA uptake can be prepared from cells harvested after exponential growth phase and subsequently treated by the CaCl₂ method using procedures well known in the art. Alternatively, MgCl₂ or RbCl can be used. Transformation can also be performed after forming a protoplast of the host cell if desired.

When the host is a eukaryote, such methods of transfection of DNA as calcium phosphate co-precipitates, conventional mechanical procedures such as microinjection, electroporation, insertion of a plasmid encased in liposomes, or virus vectors may be used. Eukaryotic cells can also be cotransformed with DNA sequences encoding the STRL33 of the invention, and a second foreign DNA molecule encoding a selectable phenotype, such as the herpes simplex thymidine kinase gene. Another method is to use a eukaryotic viral vector, such as simian virus 40 (SV40) or bovine papilloma virus, to transiently infect or transform eukaryotic cells and express the protein. (see for example, Eukaryotic Viral Vectors, Cold Spring Harbor Laboratory, Gluzman ed., 1982).

Isolation and purification of microbial expressed polypeptide, or fragments thereof, provided by the invention, may be carried out by conventional means including preparative chromatography and immunological separations involving monoclonal or polyclonal antibodies.

### STRL33 FUNCTIONAL ASSAY

In one embodiment, the invention provides an in vitro method for detecting susceptibility of a cell to HIV infection. The method includes incubating a first cell to be tested for susceptibility, with a second cell which is known to express HIV-*env*, under suitable conditions to allow fusion of the two cells (see below for an example of suitable conditions), wherein the cells are not human embryonic stem cells. Susceptibility is indicated by detecting fusion of the cells. Detection is preferably by a reporter gene, as described below for lacZ, however, other reporter means are known in the art and are discussed in the present specification under "Screen For STRL33 Blocking Agents".

The demonstrations that receptors for both CXC and CC chemokines can function as cofactors for HIV-1 entry into cells led to testing STRL33 in an assay designed to detect fusion between two cell populations: NIH 3T3 cells expressing T7 RNA polymerase, human CD4, and either STRL33 or fusin/CXCR4 or CCR5, and HeLa cells expressing Envs from HIV-1 isolates with distinct tropisms (see references in 32) and containing the Lac Z gene under control of a T7 promoter. DNAs encoding the GPCRs were introduced into NIH 3T3 cells by transfection and the DNAs encoding other proteins were introduced into cells using recombinant vaccinia viruses. Fusion between the transfected/infected NIH 3T3 and the Env-expressing cells resulted in expression of β-Gal. Envs examined included the prototypic TCL-tropic LAV and IIIB and the prototypic M-tropic ADA, SF162, Ba-L, and JR-FL. Recent data using the ADA Env show that it differs somewhat from the other M-tropic Envs in demonstrating low-level fusion with T cell lines (32), and with cells expressing fusin/CXCR4 (8 and see below). As a negative control, we used the Unc Env, a mutant protein that cannot mediate fusion due to a deletion of the gp120/gp41 cleavage site. Murine NIH/3T3 cells or human HeLa cells are coinfected with various vaccinia viruses: vTF7-3 (containing the T7 RNA polymerase gene); vCB3 (containing the human CD4 gene); vSTRL33 (containing the STRL33 gene); and vaccinia WR (a negative control). A different cell population is coinfected with various vaccinia viruses: vCB-21R (containing the *E*. *coli lacZ* gene under the transcriptional control of a T7 promoter (P_{T7}-*lacZ*) along with either vSC60 (containing the HIV-1 *env* gene (IIIB isolate)) or vCB-16 (a negative control, containing a mutant *env* gene encoding an uncleavable, nonfusogenic *unc*/*env*). The cell populations are incubated overnight at 31 °C to allow expression of the vaccinia-encoded proteins. The cells are washed and mixtures are prepared in 96-well microtiter plates. Each well contains equal numbers of the indicated pairs of T7 RNA polymerase-containing cells and *lacZ* gene-containing cells. Replicate plates are incubated for 4 hours at 37°C to allow fusion. Samples on one plate are treated with NP-40 and aliquots are assayed for β-galactosidase activity using a 96-well absorbance reader. Samples on the second plate are stained with crystal violet for syncytia analysis by light microscopy.

The results of the fusion assays are shown in Fig.3A. NIH 3T3 cells expressing CD4 plus fusin/CXCR4 fused well with cells expressing Envs from LAV and IIIB and much less well with cells expressing the ADA Env; β-Gal activity with the JR-FL and Ba-L Envs was not above the background seen with the non-fusogenic Unc Env. Cells expressing CD4 plus CCR5 fused well with cells expressing the Envs from the M-tropic variants ADA, SF162, Ba-L and JR-FL and not the Envs from LAV and IIIB. These results are consistent with previous reports (8, 11-15).

In contrast to the restricted specificities of fusin/CXCR4 and CCR5, STRL33 functioned with CD4 as a fusion cofactor for cells expressing Envs from both TCL-tropic and M-tropic variants. Negligible β-Gal activity, equivalent to levels seen using the Unc Env, was detected in fusion assays using CD4-expressing NIH 3T3 cells transfected with a control vector lacking the STRL33 cDNA insert, or in assays using NIH 3T3 cells transfected with the STRL33 cDNA but not expressing CD4.

STRL33 was also examined in stable lines of Jurkat cells that had been transfected with DNA encoding STRL33 or with a vector control and cloned by limiting dilution and hygromycin selection. The Jurkat cell lines were infected with recombinant vaccinia viruses encoding T7 RNA polymerase and CD4, and then mixed with cells that had been infected with the recombinant vaccinia virus encoding β-Gal and infected separately with recombinant viruses encoding Envs Unc, ADA, JR-FL or Ba-L. As shown in Fig.3B, the STRL33-transfected Jurkat cells could be fused with cells expressing the Envs from the M-tropic strains ADA, JR-FL and Ba-L but not with cells expressing Unc. The vector control-transfected Jurkat cells did not support fusion with these Envs. When the TCL-tropic LAV Env was examined, comparable levels of fusion were observed for the STRL33-transfected and the vector control-transfected cells, presumably because Jurkat cells express fusin/CXCR4 (see Fig. 2A).

Preferably, in the fusion method of the invention, the first or the second cell contains a reporter means and at least the test cell, or first cell, is a T cell. A first or second cell typically includes a T-cell for *in vivo* use and NIH-3T3 cells or any of the cells described in the following section for use *in vitro.* The fusion method described herein is also particularly useful for screening fusion inhibiting compositions, including fusion inhibiting agents and pharmacological agents, useful in treatment of HIV infection, both prophylactically and after infection. Examples of these agents are described in more detail below, and include but are not limited to peptides, antibodies, peptidomimetics, and chemical compounds.

### Cell Lines

In one embodiment, the present invention provides human and nonhuman cell lines, the cells of which contain DNA encoding STRL33 and coexpress human CD4 and STRL33. All cells used within this invention are not human embryonic stem cells. The cells which provide the starting material in which STRL33 are expressed must be STRL33 negative, but can be either CD4 positive or CD4 negative cells. Suitable cell types include but are not limited to, cells of the following types: NIH-3T3 murine fibroblasts, quail QT6 quail cells, canine Cf2Th thymocytes, MV1 Lu mink lung cells, Sf9 insect cells, primary T-cells, and human T-cell lines such as H9, U-87 MG glioma cell, and CEM. Such cells are described, for example, in the Cell Line Catalog of the American Type Culture Collection (ATCC, Rockville, MD, USA, 20852). The stable transfer of genes into mammalian cells has been well described in the art. See, for example, Ausubel et al., Introduction of DNA Into Mammalian Cells, in CURRENT PROTOCOLS IN MOLECULAR BIOLOGY, sections 9.5.1-9.5.6 (John Wiley & Sons, Inc. 1995).

STRL33 can be expressed using inducible or constituitive regulatory elements for such expression. Commonly used constituitive or inducible promoters, for example, are known in the art. The desired protein encoding sequence and an operably linked promoter may be introduced into a recipient cell either as a non-replicating DNA (or RNA) molecule, which may either be a linear molecule or, more preferably, a closed covalent circular molecule. Since such molecules are incapable of autonomous replication, the expression of the desired molecule may occur through the transient expression of the introduced sequence. Alternatively, permanent expression may occur through the integration of the introduced sequence into the host chromosome. Therefore the cells can be transformed stably or transiently.

An example of a vector that may be employed is one which is capable of integrating the desired gene sequences into the host cell chromosome. Cells which have stably integrated the introduced DNA into their chromosomes can be selected by also introducing one or more markers which allow for selection of host cells which contain the expression vector.

The marker may complement an auxotrophy in the host (such as leu2, or ura3, which are common yeast auxotrophic markers), biocide resistance, e.g., antibiotics, or heavy metals, such as copper, or the like. The selectable marker gene can either be directly linked to the DNA gene sequences to be expressed, or introduced into the same cell by co-transfection.

In a preferred embodiment, the introduced sequence will be incorporated into a plasmid or viral vector capable of autonomous replication in the recipient host. Any of a wide variety of vectors may be employed for this purpose. Factors of importance in selecting a particular plasmid or viral vector include: the ease with which recipient cells that contain the vector may be recognized and selected from those recipient cells which do not contain the vector; the number of copies of the vector which are desired in a particular host; and whether it is desirable to be able to "shuttle" the vector between host cells of different species.

For a mammalian host, several possible vector systems are available for expression. One class of vectors utilize DNA elements which provide autonomously replicating extra-chromosomal plasmids, derived from animal viruses such as bovine papilloma virus, polyoma virus, adenovirus, or SV40 virus. A second class of vectors include vaccinia virus expression vectors. A third class of vectors relies upon the integration of the desired gene sequences into the host chromosome. Cells which have stably integrated the introduced DNA into their chromosomes may be selected by also introducing one or more markers (e.g., an exogenous gene) which allow selection of host cells which contain the expression vector. The marker may provide for prototropy to an auxotrophic host, biocide resistance, e.g., antibiotics, or heavy metals, such as copper or the like. The selectable marker gene can either be directly linked to the DNA sequences to be expressed, or introduced into the same cell by co-transformation. Additional elements may also be needed for optimal synthesis of mRNA. These elements may include splice signals, as well as transcription promoters, enhancers, and termination signals. The cDNA expression vectors incorporating such elements include those described by Okayama, H., Mol. Cell. Biol., 3:280 (1983), and others.
Once the vector or DNA sequence containing the construct has been prepared for expression, the DNA construct may be introduced (transformed) into an appropriate host. Various techniques may be employed, such as protoplast fusion, calcium phosphate precipitation, electroporation or other conventional techniques.

### TRANSGENIC ANIMALS

In another embodiment, the present invention relates to transgenic non-human animals having cells that coexpress human CD4 and STRL33. Such transgenic animals represent a model system for the study of HIV infection and the development of more effective anti-HIV therapeutics. The invention also envisions transgenic animals that express other co-factors necessary for HIV-*env-*mediated cell fusion.

The term "animal" here denotes all mammalian species except human. It also includes an individual animal in all stages of development, including embryonic and fetal stages. Farm animals (pigs, goats, sheep, cows, horses, rabbits and the like), rodents (such as mice), and domestic pets (for example, cats and dogs) are included within the scope of the present invention.

A "transgenic" animal is any animal containing cells that bear genetic information received, directly or indirectly, by deliberate genetic manipulation at the subcellular level, such as by microinjection or infection with recombinant virus. "Transgenic" in the present context does not encompass classical crossbreeding or *in vitro* fertilization, but rather denotes animals in which one or more cells receive a recombinant DNA molecule. Although it is highly preferred that this molecule be integrated within the animal's chromosomes, the present invention also contemplates the use of extrachromosomally replicating DNA sequences, such as might be engineered into yeast artificial chromosomes. Transgenic animals in the sense of the present invention may also include a "germ cell line" transgenic animal. A germ cell line transgenic animal is a transgenic animal in which the genetic information has been taken up and incorporated into a germ line cell, therefore conferring the ability to transfer the information to offspring. If such offspring in fact possess some or all of that information, then they, too, are transgenic animals.

The term "transgenic animal, as used herein, does not include transgenic humans.

It is highly preferred that the transgenic animals of the present invention be produced by introducing into single cell embryos DNA encoding STRL33 and DNA encoding human CD4, in a manner such that these polynucleotides are stably integrated into the DNA of germ line cells of the mature animal and inherited in normal mendelian fashion. Advances in technologies for embryo micromanipulation now permit introduction of heterologous DNA into fertilized mammalian ova. For instance, totipotent or pluripotent stem cells can be transformed by microinjection, calcium phosphate mediated precipitation, liposome fusion, retroviral infection or other means, the transformed cells are then introduced into the embryo, and the embryo then develops into a transgenic animal. In a preferred method, developing embryos are infected with a retrovirus containing the desired DNA, and transgenic animals produced from the infected embryo. In a most preferred method, however, the appropriate DNAs are coinjected into the pronucleus or cytoplasm of embryos, preferably at the single cell stage, and the embryos allowed to develop into mature transgenic animals. Those techniques as well known. For instance, reviews of standard laboratory procedures for microinjection of heterologous DNAs into mammalian (mouse, pig, rabbit, sheep, goat, cow) fertilized ova include: Hogan et al., MANIPULATING THE MOUSE EMBRYO (Cold Spring Harbor Press 1986); Krimpenfort et al., Bio/Technology 9:86 (1991); Palmiter et al., Cell 41:343 (1985); Kraemer et al., GENETIC MANIPULATION OF THE EARLY MAMMALIAN EMBRYO (Cold Spring Harbor Laboratory Press 1985); Hammer et al., Nature, 315:680 (1985); Purcel et al., Science, 244:1281 (1986); Wagner et al., U.S. patent No. 5,175,385; Krimpenfort et al., and U.S. patent No. 5,175,384. The cDNA encoding STRL33 can be fused in proper reading frame under the transcriptional and translational control of a vector to produce a genetic construct that is then amplified, for example, by preparation in a bacterial vector, according to conventional methods. See, for example, the standard work: Sambrook et al., MOLECULAR CLONING: A LABORATORY MANUAL (Cold Spring Harbor Press 1989). The amplified construct is thereafter excised from the vector and purified for use in producing transgenic animals.

Production of transgenic animals containing the gene for human CD4 have been described. *See* Snyder *et al*., *supra* and, Dunn *et al*., *supra.*

The term "transgenic" as used herein additionally includes any organism, but not humans, whose genome has been altered by in vitro manipulation of the early embryo or fertilized egg or by any transgenic technology to induce a specific gene knockout. The term "gene knockout" as used herein, refers to the targeted disruption of a gene *in vivo* with complete loss of function that has been achieved by any transgenic technology familiar to those in the art. In one embodiment, transgenic animals having gene knockouts are those in which the target gene has been rendered nonfunctional by an insertion targeted to the gene to be rendered non-functional by homologous recombination. As used herein, the term "transgenic" includes any transgenic technology familiar to those in the art which can produce an organism carrying an introduced transgene or one in which an endogenous gene has been rendered non-functional or Aknocked out.

The transgene to be used in the practice of the subject invention is a DNA sequence comprising a modified STRL33 coding sequence. In a preferred embodiment, the STRL33 gene is disrupted by homologous targeting in non-human embryonic stem cells. For example, the entire mature C-terminal region of the STRL33 gene may be deleted as described in the examples below. Optionally, the STRL33 disruption or deletion may be accompanied by insertion of or replacement with other DNA sequences, such as a non-functional STRL33 sequence. In other embodiments, the transgene comprises DNA antisense to the coding sequence for STRL33. In another embodiment, the transgene comprises DNA encoding an antibody or receptor peptide sequence which is able to bind to STRL33. Where appropriate, DNA sequences that encode proteins having STRL33 activity but differ in nucleic acid sequence due to the degeneracy of the genetic code may also be used herein, as may truncated forms, allelic variants and interspecies homologues.

### ANTIBODIES AGAINST STRL33 INHIBIT FUSION

In another embodiment, the present invention uses antibodies against STRL33 that block *env*-mediated membrane fusion (i) associated with HIV entry into a human CD4-positive target cell or (ii) between an HIV-infected cell and an uninfected human CD4-positive target cell. Such antibodies are useful as research and diagnostic tools in the study of HIV infection and the development of more effective anti-HIV therapeutics. In addition, pharmaceutical compositions comprising antibodies against STRL33 may represent effective anti-HIV therapeutics.

A target cell typically includes a T-cell for *in vivo* use and NIH-3T3 cells or any of the above-listed cells for use *in vitro.* Antibodies used in the invention include polyclonal antibodies, monoclonal antibodies, and fragments of polyclonal and monoclonal antibodies.

The STRL33 polypeptides can also be used to produce antibodies which are immunoreactive or bind to epitopes of the STRL33 polypeptides. Antibody which consists essentially of pooled monoclonal antibodies with different epitopic specificities, as well as distinct monoclonal antibody preparations are provided. Monoclonal antibodies are made from antigen containing fragments of the protein by methods well known in the art (Kohler, et al., Nature, 256:495, 1975; Current Protocols in Molecular Biology, Ausubel, et al., ed., 1989).

The term "antibody" as used in this invention includes intact molecules as well as fragments thereof, such as Fab, F(ab')₂, and Fv which are capable of binding the epitopic determinant. These antibody fragments retain some ability to selectively bind with its antigen or receptor and are defined as follows:
(1) Fab, the fragment which contains a monovalent antigen-binding fragment of an antibody molecule can be produced by digestion of whole antibody with the enzyme papain to yield an intact light chain and a portion of one heavy chain;
(2) Fab', the fragment of an antibody molecule can be obtained by treating whole antibody with pepsin, followed by reduction, to yield an intact light chain and a portion of the heavy chain; two Fab' fragments are obtained per antibody molecule;
(3) (Fab')₂, the fragment of the antibody that can be obtained by treating whole antibody with the enzyme pepsin without subsequent reduction; F(ab')₂ is a dimer of two Fab' fragments held together by two disulfide bonds;
(4) Fv, defined as a genetically engineered fragment containing the variable region of the light chain and the variable region of the heavy chain expressed as two chains; and
(5) Single chain antibody ("SCA"), defined as a genetically engineered molecule containing the variable region of the light chain, the variable region of the heavy chain, linked by a suitable polypeptide linker as a genetically fused single chain molecule.

Methods of making these fragments are known in the art. (See for example, Harlow and Lane, Antibodies: A Laboratory Manual, Cold Spring Harbor Laboratory, New York (1988)).

As used in this invention, the term "epitope" means any antigenic determinant on an antigen to which the paratope of an antibody binds. Epitopic determinants usually consist of chemically active surface groupings of molecules such as amino acids or sugar side chains and usually have specific three dimensional structural characteristics, as well as specific charge characteristics.

Antibodies which bind to the STRL33 polypeptide can be prepared using an intact polypeptide or fragments containing small peptides of interest as the immunizing antigen. The polypeptide or a peptide used to immunize an animal can be derived from translated cDNA or chemical synthesis which can be conjugated to a carrier protein, if desired. Such commonly used carriers which are chemically coupled to the peptide include keyhole limpet hemocyanin (KLH), thyroglobulin, bovine serum albumin (BSA), and tetanus toxoid. The coupled peptide is then used to immunize the animal (*e*.*g.,* a mouse, a rat, or a rabbit).

If desired, polyclonal or monoclonal antibodies can be further purified, for example, by binding to and elution from a matrix to which the polypeptide or a peptide to which the antibodies were raised is bound. Those of skill in the art will know of various techniques common in the immunology arts for purification and/or concentration of polyclonal antibodies, as well as monoclonal antibodies (See for example, Coligan, et al., Unit 9, Current Protocols in Immunology, Wiley Interscience, 1994).

It is also possible to use the anti-idiotype technology to produce monoclonal antibodies which mimic an epitope. For example, an anti-idiotypic monoclonal antibody made to a first monoclonal antibody will have a binding domain in the hypervariable region which is the "image" of the epitope bound by the first monoclonal antibody.

The preparation of polyclonal antibodies is well-known to those skilled in the art. See, for example, Green et al., Production of Polyclonal Antisera, in IMMUNOCHEMICAL PROTOCOLS (Manson, ed.), pages 1-5 (Humana Press 1992), and Coligan et al., Production of Polyclonal Antisera in Rabbits, Rats, Mice and Hamsters, in CURRENT PROTOCOLS IN IMMUNOLOGY, section 2.4.1 (1992).

The preparation of monoclonal antibodies likewise is conventional. See, for example, Kohler & Milstein, Nature 256:495 (1975); Coligan *et al*., sections 2.5.1-2.6.7; and Harlow et al., ANTIBODIES: A LABORATORY MANUAL, page 726 (Cold Spring Harbor Pub. 1988). Briefly, monoclonal antibodies can be obtained by injecting mice with a composition comprising an antigen, verifying the presence of antibody production by removing a serum sample, removing the spleen to obtain B lymphocytes, fusing the B lymphocytes with myeloma cells to produce hybridomas, cloning the hybridomas, selecting positive clones that produce antibodies to the antigen, and isolating the antibodies from the hybridoma cultures. Monoclonal antibodies can be isolated and purified from hybridoma cultures by a variety of well-established techniques. Such isolation techniques include affinity chromatography with Protein-A Sepharose, size-exclusion chromatography, and ionexchange chromatography. *See, e*.*g*., *Coligan et al*., sections 2.7.1-2.7.12 and sections 2.9.1-2.9.3; Barnes et al., Purification of Immunoglobulin G (IgG), in METHODS IN MOLECULAR BIOLOGY, VOL. 10, pages 79-104 (Humana Press 1992). Methods of *in vitro* and *in vivo* multiplication of monoclonal antibodies is well-known to those skilled in the art. Multiplication *in vitro* may be carried out in suitable culture media such as Dulbecco's Modified Eagle Medium or RPMI 1640 medium, optionally replenished by a mammalian serum such as fetal calf serum or trace elements and growth-sustaining supplements such as normal mouse peritoneal exudate cells, spleen cells, bone marrow macrophages. Production *in vitro* provides relatively pure antibody preparations and allows scale-up to yield large amounts of the desired antibodies. Large scale hybridoma cultivation can be carried out by homogenous suspension culture in an airlift reactor, in a continuous stirrer reactor, or in immobilized or entrapped cell culture. Multiplication *in vivo* may be carried out by injecting cell clones into mammals histocompatible with the parent cells, e.g., syngeneic mice, to cause growth of antibody-producing tumors. Optionally, the animals are primed with a hydrocarbon, especially oils such as pristane (tetramethylpentadecane) prior to injection. After one to three weeks, the desired monoclonal antibody is recovered from the body fluid of the animal.

Therapeutic applications are conceivable for the antibodies of the present invention. For example, antibodies of the present invention may also be derived from subhuman primate antibody. General techniques for raising therapeutically useful antibodies in baboons may be found, for example, in Goldenberg *et al.,* International Patent Publication WO 91/11465 (1991) and Losman et al., Int. J. Cancer 46:310 (1990).

Alternatively, a therapeutically useful anti-STRL33 antibody may be derived from a "humanized" monoclonal antibody. Humanized monoclonal antibodies are produced by transferring mouse complementary determining regions from heavy and light variable chains of the mouse immunoglobulin into a human variable domain, and then substituting human residues in the framework regions of the murine counterparts. The use of antibody components derived from humanized monoclonal antibodies obviates potential problems associated with the immunogenicity of murine constant regions. General techniques for cloning murine immunoglobulin variable domains are described, for example, by Orlandi et al., Proc. Nat'l Acad. Sci. USA 86:3833 (1989). Techniques for producing humanized monoclonal antibodies are described, for example, by Jones et al., Nature 321: 522 (1986); Riechmann et al., Nature 332: 323 (1988); Verhoeyen et al., Science 239: 1534 (1988); Carter et al., Proc. Nat'l Acad Sci. USA 89: 4285 (1992); Sandhu, Crit. Rev. Biotech. 12: 437 (1992); and Singer et al., J Immunol. 150: 2844 (1993).

Antibodies also may be derived from human antibody fragments isolated from a combinatorial immunoglobulin library. See, for example, Barbas et al., METHODS: A COMPANION TO METHODS IN ENZYMOLOGY, VOL. 2, page 119 (1991); Winter et al., Ann. Rev. Immunol. 12: 433 (1994). Cloning and expression vectors that are useful for producing a human immunoglobulin phage library can be obtained, for example, from STRATAGENE Cloning Systems (La Jolla, CA).

In addition, antibodies may be derived from a human monoclonal antibody. Such antibodies are obtained from transgenic mice that have been "engineered" to produce specific human antibodies in response to antigenic challenge. In this technique, elements of the human heavy and light chain loci are introduced into strains of mice derived from embryonic stem cell lines that contain targeted disruptions of the endogenous heavy and light chain loci. The transgenic mice can synthesize human antibodies specific for human antigens, and the mice can be used to produce human antibody-secreting hybridomas. Methods for obtaining human antibodies from transgenic mice are described by Green et al., Nature Genet. 7:13 (1994); Lonberg et al., Nature 368:856 (1994); and Taylor et al., Int. Immunol. 6:579 (1994).

Antibody fragments can be prepared by proteolytic hydrolysis of the antibody or by expression in *E*. *coli* of DNA encoding the fragment. Antibody fragments can be obtained by pepsin or papain digestion of whole antibodies by conventional methods. For example, antibody fragments can be produced by enzymatic cleavage of antibodies with pepsin to provide a 5S fragment denoted F(ab')₂. This fragment can be further cleaved using a thiol reducing agent, and optionally a blocking group for the sulfhydryl groups resulting from cleavage of disulfide linkages, to produce 3.5S Fab' monovalent fragments. Alternatively, an enzymatic cleavage using papain produces two monovalent Fab fragments and an Fc fragment directly. These methods are described, for example, by Goldenberg, U.S. patents No. 4,036,945 and No. 4,331,647. *See also* Nisonhoff et al., Arch. Biochem. Biophys. 89:230 (1960); Porter, Biochem. J. 73:119 (1959); Edelman et al., METHODS IN ENZYMOLOGY, VOL. 1, page 422 (Academic Press 1967); and Coligan *et al*. at sections 2.8.1-2.8.10 and 2.10.1-2.10.4.

Other methods of cleaving antibodies, such as separation of heavy chains to form monovalent light-heavy chain fragments, further cleavage of fragments, or other enzymatic, chemical, or genetic techniques may also be used, so long as the fragments bind to the antigen that is recognized by the intact antibody.

For example, Fv fragments comprise an association of V_{H} and Y chains. This association may be noncovalent, as described in Inbar et al., Proc. Nat'l Acad. Sci. USA 69:2659 (1972). Alternatively, the variable chains can be linked by an intermolecular disulfide bond or cross-linked by chemicals such as glutaraldehyde. *See*, *e*.*g*., Sandhu, *supra.* Preferably, the Fv fragments comprise V_{H} and V_{L} chains connected by a peptide linker. These single-chain antigen binding proteins (sFv) are prepared by constructing a structural gene comprising DNA sequences encoding the V_{H} and V_{L} domains connected by an oligonucleotide. The structural gene is inserted into an expression vector, which is subsequently introduced into a host cell such as *E*. *coli*. The recombinant host cells synthesize a single polypeptide chain with a linker peptide bridging the two V domains. Methods for producing sFvs are described, for example, by Whitlow et al., METHODS: A COMPANION TO METHODS IN ENZYMOLOGY, VOL. 2, page 97 (1991); Bird et al., Science 242:423-426 (1988); Ladner et al., U.S. patent No. 4,946,778; Pack et al., Bio/Technology 11: 1271-77 (1993); and Sandhu, *supra*.

Another form of an antibody fragment is a peptide coding for a single complementarity-determining region (CDR). CDR peptides ("minimal recognition units") can be obtained by constructing genes encoding the CDR of an antibody of interest. Such genes are prepared, for example, by using the polymerase chain reaction to synthesize the variable region from RNA of antibody-producing cells. See, for example, Larrick et al., METHODS: A COMPANION TO METHODS IN ENZYMOLOGY, VOL. 2, page 106 (1991).

It is also envisioned that antibodies may block HIV-*env* mediated cell fusion or infection by blocking the interaction between CD4, STRL33 and HIV, without actually "binding" to STRL33. Therefore, all of the above descriptions regarding antibodies that bind to STRL33 also apply to antibodies that block HIV-*env* mediated infection or fusion.

### SCREEN FOR STRL33 BINDING AND BLOCKING COMPOSITIONS

In another aspect of the present invention, there is provided a method for identifying a composition which binds to STRL33 or blocks HIV env-mediated membrane fusion. The method includes incubating components comprising the composition and a recombinant host cell stably transformed with a polynucleotide encoding STRL33 polypeptide and, optionally, with a polynucleotide encoding CD4 polypeptide, wherein said recombinant host cell co-expresses STRL33 and CD4 polypeptide, and is not a human embryonic stem cell, under conditions sufficient to allow the components to interact and measuring the binding of the composition to STRL33.

Compositions that bind to STRL33 include peptides, peptidomimetics, polypeptides, chemical compounds and biologic agents as described in more detail below.

Furthermore describes peptides which bind to STRL33 may include peptide fragments of STRL33 that block membrane fusion. Such peptide fragments could represent research and diagnostic tools in the study of HIV infection and the development of more effective anti-HIV therapeutics. In addition, pharmaceutical compositions comprising isolated and purified peptide fragments of STRL33 may represent effective anti-HIV therapeutics.

For example naturally-occurring STRL33, but also STRL33 mutants and chemically synthesized derivatives of STRL33 that block membrane fusion between HIV and a target cell, could represent such research and diagnostic tools in the study of HIV infection and the development of more effective anti-HIV therapeutics. STRL33 can be altered by changing the DNA encoding the protein. Preferably, only conservative amino acid alterations are undertaken, using amino acids that have the same or similar properties. Illustrative amino acid substitutions include the changes of: alanine to serine; arginine to lysine; asparagine to glutamine or histidine; aspartate to glutamate; cysteine to serine; glutamine to asparagine; glutamate to aspartate; glycine to proline; histidine to asparagine or glutamine; isoleucine to leucine or valine; leucine to valine or isoleucine; lysine to arginine, glutamine, or glutamate; methionine to leucine or isoleucine; phenylalanine to tyrosine, leucine or methionine; serine to threonine; threonine to serine; tryptophan to tyrosine; tyrosine to tryptophan or phenylalanine; valine to isoleucine or leucine.

Additionally, the composition may include other variants and fragments of STRL33 that bind to STRL33 and which could be used as research and diagnostic tools in the study of HIV infection and the development of more effective anti-HIV therapeutics. Variants include analogs, homologs, derivatives, muteins and mimetics of STRL33 that retain the ability to block membrane fusion. Fragments of the STRL33 refer to portions of the amino acid sequence of STRL33 that also retain this ability. The variants and fragments can be generated directly from STRL33 itself by chemical modification, by proteolytic enzyme digestion, or by combinations thereof. Additionally, genetic engineering techniques, as well as methods of synthesizing polypeptides directly from amino acid residues, can be employed.

A peptide fragment which blocks membrane fusion could also be a fragment of HIV env.

Non-peptide compounds that mimic the binding and function of STRL33 ("mimetics") can be produced by the approach outlined in Saragovi et al., Science 253: 792-95 (1991). Mimetics are molecules which mimic elements of protein secondary structure. See, for example, Johnson et al.,"Peptide Turn Mimetics," in BIOTECHNOLOGY AND PHARMACY, Pezzuto et al., Eds., (Chapman and Hall, New York 1993). The underlying rationale behind the use of peptide mimetics is that the peptide backbone of proteins exists chiefly to orient amino acid side chains in such a way as to facilitate molecular interactions.

Variants and fragments also can be created by recombinant techniques employing genomic or cDNA cloning methods. Site-specific and region-directed mutagenesis techniques can be employed. See CURRENT PROTOCOLS IN MOLECULAR BIOLOGY vol. 1, ch. 8 (Ausubel et al. eds., J. Wiley & Sons 1989 & Supp. 1990-93); PROTEIN ENGINEERING (Oxender & Fox eds., A. Liss, Inc. 1987). In addition, linker-scanning and PCR-mediated techniques can be employed for mutagenesis. See PCR TECHNOLOGY (Erlich ed., Stockton Press 1989); CURRENT PROTOCOLS IN MOLECULAR BIOLOGY, vols. 1 & 2, *supra*. Protein sequencing, structure and modeling approaches for use with any of the above techniques are disclosed in PROTEIN ENGINEERING, *loc. cit*., and CURRENT PROTOCOLS IN MOLECULAR BIOLOGY, vols. 1 & 2, *supra*.

Furthermore various chemokines may function as a biologic agent as a ligand for STRL33. Derivatives, analogs, mutants and STRL33 binding fragments of STRL33 ligand may also block *env*-mediated membrane fusion.

Incubating includes conditions which allow contact between the test composition and STRL33. Binding can be measured indirectly by biochemical alterations in the cell (e.g., calcium flux). Contacting includes in solution and in solid phase. The test ligand(s)/composition may optionally be a combinatorial library for screening a plurality of compositions. Compositions identified in the method of the invention can be further evaluated, detected, cloned, sequenced, and the like, either in solution or after binding to a solid support, by any method usually applied to the detection of a specific DNA sequence such as PCR, oligomer restriction (Saiki, et al., Bio/Technology, 3:1008-1012, 1985), allele-specific oligonucleotide (ASO) probe analysis (Conner, et al., Proc. Natl. Acad. Sci. USA, 80:278, 1983), oligonucleotide ligation assays (OLAs) (Landegren, et al., Science, 241:1077, 1988), and the like. Molecular techniques for DNA analysis have been reviewed (Landegren, et al., Science, 242:229-237, 1988).

Any of a variety of procedures may be used to clone the genes of the present invention when the test composition is in a combinatorial library or is expressed as a gene product (as opposed to a chemical composition). One such method entails analyzing a shuttle vector library of DNA inserts (derived from a cell which expresses the composition) for the presence of an insert which contains the composition gene. Such an analysis may be conducted by transfecting cells with the vector and then assaying for expression of the composition binding activity. The preferred method for cloning these genes entails determining the amino acid sequence of the composition protein. Usually this task will be accomplished by purifying the desired composition protein and analyzing it with automated sequencers. Alternatively, each protein may be fragmented as with cyanogen bromide, or with proteases such as papain, chymotrypsin or trypsin (Oike, Y., et al., J. Biol. Chem., 257:9751-9758 (1982); Liu, C., et al., Int. J. Pept. Protein Res., 21:209-215 (1983)). Although it is possible to determine the entire amino acid sequence of these proteins, it is preferable to determine the sequence of peptide fragments of these molecules.

To determine if a composition can functionally complex with the receptor protein, induction of the exogenous gene is monitored by monitoring changes in the protein levels of the protein encoded for by the exogenous gene, for example. When a composition(s) is found that can induce transcription of the exogenous gene, it is concluded that this composition(s) can bind to the receptor protein coded for by the nucleic acid encoding the initial sample test composition(s).

Expression of the exogenous gene can be monitored by a functional assay or assay for a protein product, for example. The exogenous gene is therefore a gene which will provide an assayable/measurable expression product in order to allow detection of expression of the exogenous gene. Such exogenous genes include, but are not limited to, reporter genes such as chloramphenicol acetyltransferase gene, an alkaline phosphatase gene, beta-galactosidase,a luciferase gene, a green fluorescent protein gene, guanine xanthine phosphoribosyltransferase, alkaline phosphatase, and antibiotic resistance genes (e.g., neomycin phosphotransferase).

Expression of the exogenous gene is indicative of composition-receptor binding, thus, the binding or blocking composition can be identified and isolated. The compositions of the present invention can be extracted and purified from the culture media or a cell by using known protein purification techniques commonly employed, such as extraction, precipitation, ion exchange chromatography, affinity chromatography, gel filtration and the like. Compositions can be isolated by affinity chromatography using the modified receptor protein extracellular domain bound to a column matrix or by heparin chromatography.

Also included in the screening method of the invention is combinatorial chemistry methods for identifying chemical compounds that bind to STRL33. Ligands/compositions that bind to STRL33 can be assayed in standard cell:cell fusion assays, such as the vaccinia assay described herein to determine whether the composition inhibits or blocks *env*-mediated membrane fusion (i) involved in HIV entry into a human CD4-positive target cell or (ii) between an HIV-infected cell and an uninfected human CD4-positive target cell.

### Preparation of PHARMACEUTICAL COMPOSITIONS

The invention also contemplates various pharmaceutical compositions that block membrane fusion between HIV and a target cell. The pharmaceutical compositions are prepared by bringing an antibody against STRL33 or an epitope binding fragment thereof or a STRL33 specific antisense polynucleotide into a form suitable for administration (e.g., a pharmaceutically acceptable carrier) to a subject using carriers, excipients and additives or auxiliaries. Frequently used carriers or auxiliaries include magnesium carbonate, titanium dioxide, lactose, mannitol and other sugars, talc, milk protein, gelatin, starch, vitamins, cellulose and its derivatives, animal and vegetable oils, polyethylene glycols and solvents, such as sterile water, alcohols, glycerol and polyhydric alcohols. Intravenous vehicles include fluid and nutrient replenishers. Preservatives include antimicrobial, anti-oxidants, chelating agents and inert gases. Other pharmaceutically acceptable carriers include aqueous solutions, non-toxic excipients, including salts, preservatives, buffers and the like, as described, for instance, in Remington's Pharmaceutical Sciences, 15th ed. Easton: Mack Publishing Co., 1405-1412, 1461-1487 (1975) and The National Formulary XIV., 14th ed. Washington: American Pharmaceutical Association (1975). The pH and exact concentration of the various components of the pharmaceutical composition are adjusted according to routine skills in the art. *See Goodman and Gilman's The Pharmacological Basis for Therapeutics* (7th ed.).

The pharmaceutical compositions are preferably prepared and administered in dose units. Solid dose units are tablets, capsules and suppositories. For treatment of a patient, depending on activity of the compound, manner of administration, nature and severity of the disorder, age and body weight of the patient, different daily doses are necessary. Under certain circumstances, however, higher or lower daily doses may be appropriate. The administration of the daily dose can be carried out both by single administration in the form of an individual dose unit or else several smaller dose units and also by multiple administration of subdivided doses at specific intervals.

The dosage should not be so large as to cause adverse side effects, such as unwanted cross-reactions, anaphylactic reactions and the like. Generally, the dosage will vary with the age, condition, sex, and extent of the disease in the patient and can be determined by one skilled in the art. The dosage can be adjusted by the individual physician in the event of any contraindications and can be readily ascertained without resort to undue experimentation. In any event, the effectiveness of treatment can be determined by monitoring the level of CD4+ T-cells in a patient. An increase or stabilization in the relative number of CD4+ cells should correlate with recovery of the patient's immune system.

The pharmaceutical compositions are in general administered topically, intravenously, orally or parenterally or as implants, but even rectal use is possible in principle. Suitable solid or liquid pharmaceutical preparation forms are, for example, granules, powders, tablets, coated tablets, (micro)capsules, suppositories, syrups, emulsions, suspensions, creams, aerosols, drops or injectable solution in ampule form and also preparations with protracted release of active compounds, in whose preparation excipients and additives and/or auxiliaries such as disintegrants, binders, coating agents, swelling agents, lubricants, flavorings, sweeteners or solubilizers are customarily used as described above. The pharmaceutical compositions are suitable for use in a variety of drug delivery systems. For a brief review of present methods for drug delivery, see Langer, Science, 249: 1527-1533 (1990).

The pharmaceutical compositions may be administered locally or systemically. By "therapeutically effective dose" is meant the quantity of a compound according to the invention necessary to prevent, to cure or at least partially arrest the symptoms of the disease and its complications. Amounts effective for this use will, of course, depend on the severity of the disease and the weight and general state of the patient. Typically, dosages used *in vitro* may provide useful guidance in the amounts useful for *in situ* administration of the pharmaceutical composition, and animal models may be used to determine effective dosages for treatment of particular disorders. Various considerations are described, e.g., in Gilman et al. (eds.) (1990) GOODMAN AND GILMAN'S: THE PHARMACOLOGICAL BASES OF THERAPEUTICS, 8th ed., Pergamon Press; and REMINGTON'S PHARMACEUTICAL SCIENCES, 17th ed. (1990), Mack Publishing Co., Easton, Pa. Effectiveness of the dosage can be monitored by CD4+ count as described above in this section.

The medicaments including antibodies or epitope binding fragment thereof and antisense nucleic acids are all useful for treating subjects either having or at risk of having an HIV related disorder. AIDS and ARC are preferred examples of such disorders. HIV-associated disorders have been recognized primarily in "at risk" groups, including homosexually active males, intravenous drug users, recipients of blood or blood products, and certain populations from Central Africa and the Caribbean. The syndrome has also been recognized in heterosexual partners of individuals in all "at risk" groups and in infants of affected mothers.

The use of an anti-STRL33 specific antibody or epitope binding fragment thereof or a STRL33 specific antisense nucleic acid (see below) includes a prophylactic treatment directed to those hosts at risk for the HIV infection. For example, the method is useful for humans at risk for HIV infection. A "prophylactically effective" amount of antibody or peptide, for example, refers to that amount which is capable of blocking *env*-mediated membrane fusion in HIV entry into a human CD4-positive target cell or between an HIV-infected cell and an uninfected human CD4-positive target cell.

Transmission of HIV occurs by at least three known routes: sexual contact, blood (or blood product) transfusion and via the placenta. Infection via blood includes transmission among intravenous drug users. Since contact with HIV does not necessarily result in symptomatic infection, as determined by seroconversion, all humans may be potentially at risk and, therefore, should be considered for prophylactic treatment by the therapeutic method of the invention.

The medicaments described herein can be administered to a patient prior to infection with HIV (i.e., prophylactically) or at any of the stages described below, after initial infection. The HIV infection may run any of the following courses: 1) approximately 15% of infected individuals have an acute illness, characterized by fever, rash, and enlarged lymph nodes and meningitis within six weeks of contact with HIV. Following this acute infection, these individuals become asymptomatic. 2) The remaining individuals with HIV infection are not symptomatic for years. 3) Some individuals develop persistent generalized lymphadenopathy (PGL), characterized by swollen lymph nodes in the neck, groin and axilla. Five to ten percent of individuals with PGL revert to an asymptomatic state. 4) Any of these individuals may develop AIDS-related complex (ARC); patients with ARC do not revert to an asymptomatic state. 5) Individuals with ARC and PGL, as well as asymptomatic individuals, eventually (months to years later) develop AIDS which inexorably leads to death.

### GENE THERAPY

This approach employs, for example, antisense nucleic acids (i.e., nucleic acids that are complementary to, or capable of hybridizing with, a target nucleic acid, e.g., a nucleic acid encoding a STRL33 polypeptide), ribozymes, or triplex agents. The antisense and triplex approaches function by masking the nucleic acid, while the ribozyme strategy functions by cleaving the nucleic acid. In addition, antibodies that bind to STRL33 polypeptides can be used in methods to block the entry of HIV into a cell or block cell fusion between HIV infected and uninfected cells.

The use of antisense methods to inhibit the *in vitro* translation of genes is well known in the art (see, e.g., Marcus-Sakura, Anal. Biochem., 172:289, 1988). Antisense nucleic acids are nucleic acid molecules (*e*.*g*., molecules containing DNA nucleotides, RNA nucleotides, or modifications (*e*.*g*., modification that increase the stability of the molecule, such as 2'-O-alkyl (*e*.*g*., methyl) substituted nucleotides) or combinations thereof) that are complementary to, or that hybridize to, at least a portion of a specific nucleic acid molecule, such as an RNA molecule (*e*.*g*., an mRNA molecule) (see, *e*.*g*., Weintraub, Scientific American, 262:40, 1990). The antisense nucleic acids hybridize to corresponding nucleic acids, such as mRNAs, to form a double-stranded molecule, which interferes with translation of the mRNA, as the cell will not translate an double-stranded mRNA. Antisense nucleic acids used in the invention are typically at least 10-12 nucleotides in length, for example, at least 15, 20, 25, 50, 75, or 100 nucleotides in length. The antisense nucleic acid can also be as long as the target nucleic acid with which it is intended that it form an inhibitory duplex. As is described further below, the antisense nucleic acids can be introduced into cells as antisense oligonucleotides, or can be produced in a cell in which a nucleic acid encoding the antisense nucleic acid has been introduced by, for example, using gene therapy methods.

In addition to blocking mRNA translation, oligonucleotides, such as antisense oligonucleotides, can be used in methods to stall transcription, such as the triplex method. In this method, an oligonucleotide winds around double-helical DNA in a sequence-specific manner, forming a three-stranded helix, which blocks transcription from the targeted gene. These triplex compounds can be designed to recognize a unique site on a chosen gene (Maher, et al., Antisense Res. and Dev., 1(3):227, 1991; Helene, Anticancer Drug Design, 6(6):569, 1991). Specifically targeted ribozymes can also be used in therapeutic methods directed at decreasing STRL33 expression.

Introduction of STRL33 antisense nucleic acids into cells affected by a proliferative disorder, for the purpose of gene therapy, can be achieved using a recombinant expression vector, such as a chimeric virus or a colloidal dispersion system, such as a targeted liposome. Those of skill in this art know or can easily ascertain the appropriate route and means for introduction of sense or antisense STRL33 nucleic acids, without resort to undue experimentation.

### HOMOZYGOUS AND HETEROZYGOUS MUTATIONS IN STRL33

It is known that in some cases, a homozygous or heterozygous mutation in a polypeptide or a regulatory region of a gene confers a molecular basis for a difference in function. Bertina, *et al*. and Greengard, *et al*. (Bertina, et al., Nature, 369:64, 1994; Greengard, et al., Lancet, 343:1361, 1994), first identified the molecular basis for the FV abnormality. The phenotype of APC resistance was shown to be associated with heterozygosity or homozygosity for a single point mutation in the FV gene that resulted in the substitution of arginine at amino acid residue 506 with glutamine (FV R506Q).

This R506Q mutation prevents APC from cleaving a peptide bond at Arg-506 in FV that is required to inactivate factor Va (Bertina, *supra*; Sun, et al., Blood, 83:3120, 1994).

Similarly, the present invention envisions diagnostic and prognostic, and in addition, therapeutic approaches to treatment of HIV-associated syndromes based on homozygosity or heterozygosity of STRL33 mutants. For example, while not wanting to be bound by a particular theory, it is believed that a subject having a homozygous mutant of STRL33 may be HIV resistant or exhibit a slower rate of disease progression. Along the same lines, a subject having a heterozygous mutation in STRL33 may exhibit a slower rate of disease progression than a patient having a wild type STRL33. Mutations included in the STRL33 coding region may also result in inactivating mutations. In addition, a mutation in the regulatory region of STRL33 gene may prevent or inhibit expression of STRL33, thereby providing resistance to some degree from HIV infection.

As described above, polymorphisms were identified in the ORF of STRL33. Specifically, a clone was identified as having second position change in the 25th codon (GAC-GCC) results in a substitution of aspartic acid with alanine. A silent change was also identified in codon 103.

Once an individual having a homozygous or heterozygous mutant in STRL33 is identified, it is envisioned that cells from that individual, once matched for histocompatibility, can be transplanted to an HIV positive individual, or to an "at risk" individual.

### EXAMPLES

### MATERIALS AND METHODS

Cell Culture. Jurkat, SUP-T1, U937, human embryonic kidney (HEK) 293, HeLa and NIH 3T3 cells were obtained from ATCC. CEM clone 12D7 was obtained from Dr. Keith Peden (CBER, FDA). Tumor infiltrating lymphocytes (TIL) R4, R8, F9 and B10, prepared from human melanomas, were obtained from Dr. John R. Yannelli, National Cancer Institute. EBV414 is an EBV-transformed B lymphoblastoid cell line obtained from Dr. Robert Siliciano, Johns Hopkins University. Jurkat, SUP-T1, CEM, U937 and EBV414 cells were grown in RPMI 1640 with 10% FBS. 293 cells were grown in MEM plus 10% horse serum. HeLa and NIH 3T3 cells were grown in DMEM with 10% FBS. TIL were grown in either RPMI 1640 with 10% FBS or in AIM-V (Life Technologies), in each case supplemented with 500 U/ml IL-2 and the cells were stimulated periodically with 250 ng/ml PHA plus irradiated allogeneic PBMC. Granulocytes were prepared as described and elutriated PBL and monocytes were prepared from normal donors by the Department of Transfusion Medicine, NIH.

Cloning of STRL33 cDNAs. Total RNA was prepared from the F9 TIL using TRIzol reagent (Life Technologies), poly(A)+ RNA was selected using oligo(dT) cellulose (Collaborative Biomedical Products), and first strand cDNA was synthesized using oligo(dT) primers and the SuperScript Preamplification System (Life Technologies) according to suppliers' protocols. For amplification, primer pools were designed based on transmembrane domain (TMD) II and TMD VII amino acid sequences from the human sequences for IL8RA, IL8RB, CCR1 and CCR2 and the murine homologues of IL8RandCCR1 and were 5'GA(T/C)(C/T)TI(C/T/G)TITT(T/C)(G/T/C)(C/T) I (T/C/A)TIACI(T/C)TICC, and 5'CCIA(T/C)(A/G)AAI (G/A)(C/T)(A/G) TAIA(T/A/G)IA(G/A/T/C)IGG(A/G)TT, respectively. Amplifications were done with cDNA synthesized from 0.015 mg of Poly(A)+ RNA, with 1.5 mM of each primer pool in a 20 ml reaction volume with Taq polymerase and reagents from Perkin Elmer according to the supplier's protocol. PCR was done using 30 cycles of denaturation at 94oC for 0.5 min, annealing at 45oC for 2 min and chain extension at 72oC for 1.5 min.

One µl from the first PCR was used in a second PCR done identically to the first and the products of the second reaction were separated on a 1.5% agarose gel from which fragments of the approximate predicted size of 670 bp were purified and inserted by blunt end ligation into the vector pNOTA/T7 (5 Prime - 3 Prime Inc.). Eighty eight ampicillin resistant bacterial transformants were picked and, to eliminate known sequences, hybridizations were done with radiolabelled oligonucleotide probes for receptors CCR1, CCR2, CCR3, fusin/CXCR4, BLR1, EBI1 and STRL22. Among the inserts in the non-hybridizing colonies was a novel sequence designated STRL33.

Using poly(A)+ RNA from F9 TIL a Lambda ZAP Express (Stratagene) cDNA library was prepared according to the supplier's protocol. 1.4x10⁶ recombinant phage from the non-amplified library were screened using a radiolabelled STRL33 probe. Ten positive phage were plaque-purified and the pBK-CMV (Stratagene) plasmids containing STRL33 inserts were recovered by in vivo excision according to the supplier's protocol. Manual and/or automated dideoxy sequencing was done for the entire cDNA clone STRL33.1, the 5' non-translated region of clone STRL33.2, the 5' non-translated region and open reading frame (ORF) of clone STRL33.3, and portions of other cDNA clones, some of which were obtained using RT-PCR.

Northern Blot Analysis. Total RNA was prepared as above. DNAs used for probes were : IL8RA, IL8RB, CCR3, EBI1 and BLR1 genomic fragments and CCR2B cDNA obtained from Dr. Philip Murphy, National Institute of Allergy and Infectious Diseases; STRL33, CCR1, CCR4, CCR5, CXCR4 and CMKBRL1 cDNAs that we isolated either from our lambda library or by RT-PCR from TIL mRNA; and an STRL22 genomic fragment isolated as described. Hybridizations to leukocyte RNA were performed as described with washes in 0.1X SSC, 0.1% SDS at 50oC . Hybridizations with an oligonucleotide probe to 18S rRNA were as described. The blot of poly(A)+ RNA from human tissues was obtained from Clontech (Palo Alto, CA). Hybridizations were done according to the supplier's protocol with washes as described above. Autoradiography/fluorography was done using an intensifying screen.

Production and analysis of STRL33-transfected cell lines. An EcoR I-Ear I fragment containing the complete STRL33 ORF was isolated from the pBK-CMV/STRL33.1 plasmid and inserted into pCEP4 (Invitrogen) and pCIneo (Promega Corp.). The pCEP4/STRL33 DNA, pCEP4 without a cDNA insert, and the pCIneo/STRL33 DNA were transfected into HEK 293 cells by calcium phosphate precipitation and into Jurkat cells by electroporation. Selection was in 200 µg/ml hygromycin B (Sigma) and 1mg/ml G418 (Life Technologies) for pCEP4 transfected cells and pCIneo transfected cells respectively. Individual colonies of resistant 293 cells were cloned and expanded and Jurkat lines were derived by limiting dilution after the electroporation. Lines expressing the highest levels of STRL33 mRNA were used to test responses to chemokines using the fluorometric calcium flux assay as described. Recombinant HuMig was obtained by infecting High Five cells of Trichoplusia ni (Invitrogen), as will be described elsewhere, and was purified by column chromatography as described . IP-10, MCP-1, MCP-2, MCP-3, RANTES, MIP-1a, MIP-1b, Platelet factor 4, IL-8, and lymphotactin, were purchased from Pepro Tech. MCP-4 was a gift from Dr. Andrew Luster, Harvard University. 1309 and SDF-1 were gifts from R & D Systems Co.

Assays for activity of STRL33 as a fusion cofactor. Assays were done using an E. coli lacZ reporter gene assay for fusion between two cell populations, one expressing an HIV-1 Env and the other expressing CD4 . Using DOTAP lipofectin (Boehringer Mannheim), NIH 3T3 cells were transfected with 10 µg of DNA, either pCIneo (Promega Corp.) containing the complete STRL33 ORF inserted downstream of the T7 promoter, or pCIneo lacking STRL33, or pCDNA3-fusin/CXCR4 or pGA9-CKR5 (encoding CCR5)(11). After 4-5 hours, the transfected cells were infected at 10 pfu/cell with recombinant vaccinia viruses vCB-3 encoding human CD4 and vTF7-3 encoding T7 RNA polymerase . A separate population of HeLa cells was co-infected with vaccinia virus vCB-21R-Lac Z containing Lac Z encoding β-galactosidase (β-Gal), under control of a T7 promoter and one of the following Env-encoding vaccinia viruses: vCB-41 encoding the LAV Env , vCB-39 encoding the ADA Env , vCB-28 encoding the JR-FL Env , vCB-32 encoding the SF-162 Env , vCB-43 encoding the Ba-L Env , vSC60 encoding the IIIB Env (S. Chakrabarti and B. Moss, personal communication), and vCB-16 encoding the non-fusogenic Unc Env . Infected cells were incubated overnight at 31oC. Duplicate samples of 10⁵ transfected and infected NIH 3 T3 target cells and 10⁵ infected Env-expressing cells were mixed; after 2.5 h cells were lysed and b-Gal activity was measured as described .

Similar assays were performed to detect Env-mediated fusion with Jurkat cell lines that had been derived, as described above, following transfection with STRL33 sequences. Jurkat cell line JC3.9 transfected with pCEP4 containing the STRL33.1 cDNA and Jurkat cell line JC0.1 transfected with pCEP4 lacking STRL33 were infected with vaccinia virus vTF7-3 (encoding T7 RNA polymerase) and VCB-3 (encoding human CD4). Following overnight incubation, the infected cells were mixed with Env-expressing HeLa cells for analyzing fusion as described above.

### REFERENCES

1. Savarese, T.M., and C.M. Fraser. 1992. In vitro mutagenesis and the search for structure-function relationships among G protein-coupled receptors. Bioch. J. 283:1-19.
2. Murphy, P. 1994. The molecular biology of leukocyte chemoattractant receptors. Ann. Rev. Immunol. 12:593-633.
3. Schall, T.J., and K. Bacon. 1994. Chemokine, leukocyte trafficking and inflammation. Curr. Opin. Immunol. 6:865-873.
4. Raport, C.J., V.L. Schweickart, D. Chantry, R.L.J. Eddy, T. Shows, R. Godiska, and P. W. Gray. 1996. New members of the chemokine receptor gene family. J. Leuk. Biol. 59:18-23.
5. Miedema, F., C. Meyaard, M. Koot, M.R. Klein, M.T. Roos, M. Groenink, R.A. Fouchier, and A. Van't Wout. 1994. Changing virus-host interactions in the course of HIV-1 infection. Immunol. Rev. 140:35-72.
6. Goudsmit, J. 1995. The role of viral diversity in HIV pathogenesis. J. Acq. Immun. Defic. Synd. Hum. R. 10:S15-S19.
7. Cocchi, F., A.L. DeVico, A. Garzino-Demo, S.K. Arya, R.C. Gallo, and P. Lusso. 1995. Identification of RANTES, MIP-1a, and MIP-1b as the major HIV-suppressive factors produced by CD8+ T cells. Science 270:1811-1815.
8. Feng, Y., C.C. Broder, P.E. Kennedy, and E.A. Berger. 1996. HIV-1 entry cofactor: functional cDNA cloning of a seven-transmembrane, G-protein-coupled receptor. Science 272:872-877.
9. Bleul, C.C., M. Farzan, H. Choe, C. Parolin, I. Clark-Lewis, J. Sodroski, and T.A. Springer. 1996. The lymphocyte chemoattractant SDF-1 is a ligand for LESTER/fusin and blocks HIV-1 entry. Nature 382:829-833.
10. Oberlin, E., A. Amara, F. Bachelerie, C. Bessia, J.-L. Virelizier, F. Arenzana-Seisdedos, O. Schwartz, J.-M. Heard, I. Clark-Lewis, D.F. Legler, M. Loetscher, M. Baggiolini, and B. Moser. 1996. The CXC chemokine SDF-1 is the ligand for LESTR/fusin and prevents infection by T-cell-line-adapted HIV-1. Nature 382:833-835.
11. Alkhatib, G., C. Combadiere, C.C. Broder, Y. Feng, P.E. Kennedy, P.M. Murphy, and E.A. Berger. 1996. CC CKR5: A RANTES, MIP-1a, MIP-1b receptor as a fusion cofactor for macrophage-tropic HIV-1. Science 272:1955-1958.
12. Deng, H., R. Liu, W. Ellmeier, S. Choe, D. Unutmaz, M. Burkhart, P. Di Marzio. S. Marmon, R.E. Sutton, C.M. Hill, C.B. Davis, S.C. Peiper, T.J. Schall, D.R. Littman, and N.R. Landau. 1996. Identification of a major co-receptor for primary isolates of HIV-1. Nature 381:661-666.
13. Choe, H., M. Farzan, Y. Sun, N. Sullivan, B. Rollins, P.D. Ponath, L. Wu, C.R. Mackay, G. LaRosa, W. Newman, N. Gerard, C. Gerard, and J. Sodroski. 1996. The b-chemokine receptors CCR3 and CCR5 facilitate infection by primary HIV-1 isolates. Cell 85:1135-1148.
14. Doranz, B.J., J. Rucker, Y. Yi, R.J. Smyth, M. Samson, S.C. Peiper, M. Parmentier, R.G. Collman, and R.W. Doms. 1996. A dual-tropic primary HIV-1 isolate that uses fusin and the b-chemokine receptors CKR-5, CKR-3, and CKR-2b as fusion cofactors. Cell 85:1149-1158.
15. Dragic, T., V. Litwin, G.P. Allaway, S.R. Martin, Y. Huang, K.A. Nagashima, C. Cayanan, P.J. Maddon, R.A. Koup, J.P. Moore, and W.A. Paxton. 1996. HIV-1 entry into CD4+ cells is mediated by the chemokine receptor CC-CKR-5. Nature 381:667-673.
16. Simmons, G., D. Wilkinson, J.D. Reeves, M.T. Dittmar, S. Beddews, J. Weber, G. Carnegie, U. Desselberger, P.W. Gray, R.A. Weiss, and P.R. Calpham. 1996. Primary, syncytium-inducing human immunodeficiency virus type I isolates are dual-tropic and most can use either Lestr or CCR-5 as co-receptors for virus entry. J. Virol. 70:8355-8360.
17. Samson, M., F. Libert, B.J. Doranz, J. Rucker, C. Liesnard, C.-M. Farber, S. Saragosti, C. Lapoumeroulie, J. Cognaux, C. Forceille, G. Muyldermans, C. Verhofstede, G. Burtonboy, M. Georges, T. Imai, S. Rana, Y. Yi, R.J. Smyth, R.G. Collman, R.W. Doms, G. Vassart, and M. Parmentier. 1996. Resistance to HIV-1 infection in caucasian individuals bearing mutant alleles of the CCR-5 chemokine receptor gene. Nature 382:722-725.
18. Liu, R., W.A. Paxton, S. Choe, D. Ceradini, S.R. Martin, R. Horuts, M.E. MacDonald, H. Stahlmann, R.A. Koup, and N.R. Candau. 1996. Homozygous defect in HIV-1 coreceptor accounts for resistance of some multiply-exposed individuals to HIV-1 infection. Cell 86:367-377.
19. Dean, M., M. Carrington, C. Winkler, G.A. Huttley, M.W. Smith, R. Allikmets, J.J. Goedert, S.P. Buchbinder, E. Vittinghoff, E. Gomperts, S. Donfield, D. Vlahov, R. Kaslow, A. Saah, C. Rinaldo, R. Detels, M.A.C.S. Hemophilia Growth and Development Study, Multicenter Hemophilia Cohort Study, A.S. San Francisco City Cohort, and S.J. O'Brien. 1996. Genetic restriction of HIV-1 infection and progression to AIDS by a deletion allele of the CKR5 structural gene. Science 273:1856-1862.
20. Zimmerman, P.A., A. Buckler-White, G. Alkhatib, T. Spalding, J. Kubofcik, C. Combadiere, D. Weissman, O. Cohen, A. Rubbert, G. Lam, M. Vaccarezza, P.E. Kennedy, V. Kumraraswami, J.V. Gorgi, R. Detels, J. Hunter, M. Chopek, E.A. Berger, A.S. Fauci, T.B. Nutman, and P.M. Murphy. 1996. Inherited resistance to HIV-1 conferred by an inactivating mutation in CC chemokine receptor 5: studies in populations with contrasting clinical phenotypes, defined racial background and quantified risk. Molecular Medicine . In press.
21. Clark, R.A., and W.M. Nauseef. 1991. Isolation and functional analysis of neutrophils. In Current protocols in Immunology. J.E. Coligan, A.M. Kruisbeek, D.H. Margulies, E.M. Shevach, and W. Strober. John Wiley and Sons, New York. 7.23.1-7.23.3.
22. Liao, F., H.-H. Lee, and J.M. Farber. 1996. Cloning of STRL22, a new human gene encoding a G protein-coupled receptor related to chemokine receptors and located on chromosome 6q27. Genomics. In press.
23. Vanguri, P., and J. Farber. 1990. Identification of CRG-2: An interferon-inducible mRNA predicted to encode a murine monokine. J. Biol. Chem. 265:15049-15057.
24. Amichay, D., R.T. Gazzinelli, G. Karupiah, T.R. Moench, A. Sher, and J.M. Farber. 1996. The gene for chemokines MuMig amd Crg-2 are induced in protozoan and viral infections in response to IFN-g with patterns of tissue expression that suggest nonredundant roles in vivo. J. Immunol. 157:4511-4520.
25. Kingston, R.E. 1996. Transfection of DNA into eukaryotic cells. In Current Protocol in Molecular Biology. R.M. Ausubel, R. Brent, R.E. Kingston, D. D. Moore, J.G.Seidman, J.A. Smith, and K, Struhl. John Wiely and Sons, New York. 9.11-9.19.9.11-9.19.
26. Memon, S.A., D. Petrak, M.B. Moreno, and C.M. Zacharchuk. 1995. A simple assay for examining the effect of transiently expressed genes on programmed cell death. J. Immunol. Methods 180:15-24.
27. Liao, F., R.L. Rabin, J.R. Yannelli, L.G. Koniaris, P. Vanguri, and J.M. Farber. 1995. Human Mig chemokine: biochemical and functional characterization. J. Exp. Med. 182:1301-1314.
28. Nussbaum, O., C.C. Broder, and E.A. Berger. 1994. Fusogenic mechanisms of enveloped-virus glycoproteins analyzed by a novel recombinant vaccinia virus-based assay quantitating cell fusion-dependent reporter gene activation. J. Virol. 68:5411-5422.
29. Broder, C.C., D.S. Dimitrov, R. Blumenthal, and E.A. Berger. 1993. The block to HIV-1 envelope glycoprotein-mediated membrane fusion in animal cells expressing human CD4 can be overcome by a human cell component(s). Virology 193:483-491.
30. Fuerst, T.R., E.G. Niles, F.W. Studier, and B. Moss. 1986. Eukaryotic transient-expression system based on recombinant vaccinia virus that synthesized bacteriophage T7 RNA polymerase. Proc. Natl. Acad. Sci. USA 83:8122-8126.
31. Alkhatib, G., C.C. Border, and E.A. Berger. 1996. Cell type-specific cofactors determine human immunodeficiency virus type I tropism for T-cell lines versus primarily macrophages. J. Virol. 70:5478-5494.
32. Broder, C.C., and E.A. Berger. 1995. Fusogenic selectivity of the envelope glycoprotein is a major determinant of human immunodeficiency virus type 1 tropism for CD4+ T-cell lines vs. primary macrophages. Proc. Natl. Acad. Sci. USA 92:9004-9008.
33. Kozak, M. 1987. An analysis of 5'-noncoding sequences from 699 vertebrate messenger RNAs. Nuc. Acid. Res. 15:8125-8148.
34. Altschul, S.F., W. Gish, W. Miller, E.W. Mayers, and D.J. Lipman. 1990. Basic local alignment search tool. J. Mol. Biol. 215:403-410.
35. Birkenbach, M., K. Josefsen, R. Yalamanchili, G. Lenoir, and E. Kieff. 1993. Epstein-Barr virus-induced genes: first lymphocyte-specific G protein-coupled peptide receptors. J. Virol. 67:2209-2220.

## Claims

1. A recombinant host cell stably transformed with a polynucleotide encoding STRL33 polypeptide, wherein the cell co-expresses STRL33 and CD4 polypeptide, and wherein the cell is not a human embryonic cell.

2. A recombinant host cell stably transformed with a polynucleotide encoding STRL33 polypeptide and a polynucleotide encoding CD4 polypeptide, wherein the cell co-expresses STRL33 and CD4 polypeptide, and wherein the cell is not a human embryonic cell.

3. The cell as in any of claims 1 and 2, wherein the cell is a human cell.

4. The cell as in any of claims 1 and 2, wherein the cell is a non-human cell.

5. Use of an anti-STRL33 specific antibody or epitope binding fragment thereof in the production of a medicament for inhibiting membrane fusion between Human Immunodeficiency Virus (HIV) and a STRL33 positive target cell or between an HIV-infected cell and a CD4 positive STRL33 positive uninfected cell wherein the anti-STRL33 specific antibody or epitope binding fragment thereof inhibits membrane fusion between HIV and a STRL33 positive target cell or between an HIV-infected cell and a CD4 positive STRL33 positive uninfected cell, and wherein the cell is not a human embryonic cell.

6. The use of claim 5, wherein the antibody is a monoclonal antibody or a polyclonal antibody.

7. The use of claim 5, wherein the anti-STRL33 specific antibody is suitable for administration by intravenous, intra-muscular or subcutaneous injections.

8. The use of claim 5, wherein the anti-STRL33 specific antibody is suitable for administration within a dose range of 0.1 µg/kg to 100 mg/kg.

9. The use of claim 5, wherein the antibody is formulated in a pharmaceutically acceptable carrier.

10. A method for identifying a composition which blocks membrane fusion between HIV and a STRL33 positive target cell or between an HIV-infected cell and a STRL33 positive uninfected cell comprising:
a) incubating components comprising the composition and the cell of claim 1 or 2 under conditions sufficient to allow the components to interact;
b) contacting the components of step a) with HIV or an HIV infected cell; and
c) measuring the ability of the composition to block membrane fusion between HIV and the STRL33 positive cell or between an HIV-infected cell and a STRL33 positive uninfected cell, wherein the cells are not human embryonic cells.

11. The method of claim 10, wherein measuring the ability of the composition to block membrane fusion is by detection of a reporter means.

12. The method of claim 11, wherein the reporter means is selected from the group consisting of a radioisotope, a fluorescent compound, a bioluminescent compound, a chemiluminescent compound, a metal chelator, or an enzyme.

13. The method of claim 12, wherein the reporter means is a lacZ gene.

14. A transgenic non-human animal having a phenotype **characterized by** expression of STRL33 polypeptide and CD4 polypeptide otherwise not naturally occurring in the animal, the phenotype being conferred by a transgene contained in the somatic and germ cells of the animal, the transgene comprising a nucleic acid sequence which encodes STRL33 polypeptide and a nucleic acid sequence which encodes CD4 polypeptide.

15. The transgenic non-human animal of claim 14, wherein the animal is a mouse.

16. The transgenic non-human animal of claim 14, wherein the animal is a rabbit.

17. A method for producing a transgenic non-human animal having a phenotype **characterized by** expression of STRL33 polypeptide and CD4 polypeptide otherwise not naturally occurring in the animal, the method comprising:
(a) introducing at least one transgene into a zygote of an animal, the transgene(s) comprising a DNA construct encoding STRL33 and a DNA construct encoding CD4,
(b) transplanting the zygote into a pseudopregnant animal,
(c) allowing the zygote to develop to term, and
(d) identifying at least one transgenic offspring containing the transgene.

18. The method of claim 17, wherein the introducing of the transgene into the embryo is by introducing an embryonic stem cell containing the transgene into the embryo.

19. The method of claim 17, wherein the introducing of the transgene into the embryo is by infecting the embryo with a retrovirus containing the transgene.

20. The method of claim 17, wherein the animal is selected from the group consisting of a mouse and a rabbit.

21. A transgenic non-human animal having a transgene disrupting or interfering with expression of STRL33 chromosomally integrated into the germ cells of the animal.

22. The transgenic animal of claim 21, wherein the animal is selected from the group consisting of a mouse and a rabbit.

23. The transgenic non-human animal of claim 22, wherein the transgene comprises a STRL33 specific antisense polynucleotide.

24. Use of an anti-STRL33 specific antibody or epitope binding fragment thereof, or a STRL33 specific antisense nucleic acid in the production of a medicament for treating a subject having or at risk of having an HIV infection or HIV-related disorder, wherein the anti-STRL33 specific antibody or epitope binding fragment thereof, or the STRL33 specific antisense nucleic acid inhibits cell-cell fusion in STRL33 positive cells infected with HIV, and wherein the cells are not human embryonic cells.

25. The use of claim 24, wherein the antibody is a monoclonal antibody.

26. The use of claim 25, wherein the monoclonal antibody is a humanized monoclonal antibody.

27. The use of claim 25, wherein the monoclonal antibody is suitable for administration to a patient suffering from Acquired Immune Deficiency syndrome (AIDS) or AIDS Related Complex (ARC).

28. The use of claim 25, wherein the monoclonal antibody is suitable for administration within a dose range between about 0.1 µg/kg to about 100 mg/kg.

29. The use of claim 25, wherein the monoclonal antibody is formulated in a pharmaceutically acceptable carrier.

30. The use of claim 29, wherein the carrier is a vector.

31. The use of claim 24, wherein the medicament is suitable for administration ex vivo.

32. An in vitro method for detecting the susceptibility of a cell to HIV infection comprising:
incubating a first cell in vitro with a second cell which expresses HIV-env under conditions to allow fusion of the two cells; and
detecting fusion of the cells, wherein fusion is indicative of the presence of STRL33 on the first cell and the susceptibility to HIV. infection, and wherein the cells are not human embryonic cells.

33. The method of claim 32, wherein the first or second cell further comprises a reporter means for detection of cell fusion.

34. The method of claim 32, wherein the first cell is a T cell.

35. The method of claim 33, wherein the reporter means is selected from the group consisting of a radioisotope, a fluorescent compound, a bioluminescent compound, a chemiluminescent compound, a metal chelator, or an enzyme.

36. The method of claim 35, wherein the reporter means is a lacZ gene.

37. An in vitro method for determining the susceptibility of a cell to HIV infection comprising detecting the presence of STRL33 on the cell, wherein the presence of STRL33 is indicative of susceptibility to HIV infection, and wherein the cell is not a human embryonic cell.

## Patentansprüche

1. Rekombinante Wirtszelle, die stabil mit einem Polynukleotid transformiert ist, das ein STRL33-Polypeptid kodiert, wobei die Zelle STRL33 und ein CD4-Polypeptid koexprimiert und wobei die Zelle keine humane embryonale Zelle ist.

2. Rekombinante Wirtszelle, die stabil mit einem ein STRL33-Polypeptid kodierendem Polynukleotid und einem ein CD4-Polypeptid kodierendem Polynukleotid transformiert ist, wobei die Zelle STRL33 und das CD4-Polypeptid koexprimiert und wobei die Zelle keine humane embryonale Zelle ist.

3. Zelle nach einem der Ansprüche 1 und 2, wobei die Zelle eine humane Zelle ist.

4. Zelle nach einem der Ansprüche 1 und 2, wobei die Zelle eine nicht-humane Zelle ist.

5. Verwendung eines spezifischen Anti-STRL33-Antikörpers oder eines Epitop-Bindungsfragments davon zur Herstellung eines Arzneimittels für die Inhibierung der Membranfusion zwischen dem humanen Immundefizienz-Virus (HIV) und einer STRL33-positiven Zielzelle oder zwischen einer HIV-infizierten Zelle und einer CD4-positiven, STRL33-positiven nicht infizierten Zelle, wobei der spezifische Anti-STRL33-Antikörper oder ein Epitop-Bindungsfragment davon die Membranfusion zwischen HIV und einer STRL33-positiven Zielzelle oder zwischen einer HIV-infizierten Zelle und einer CD4-positiven, STRL33-positiven, nicht infizierten Zelle inhibiert und wobei die Zelle keine humane embryonale Zelle ist.

6. Verwendung nach Anspruch 5, wobei der Antikörper ein monoklonaler Antikörper oder ein polyklonaler Antikörper ist.

7. Verwendung nach Anspruch 5, wobei der spezifische Anti-STRL33-Antikörper geeignet ist für eine Verabreichung mittels intravenöser, intramuskulärer oder subkutaner Injektionen.

8. Verwendung nach Anspruch 5, wobei der spezifische Anti-STRL33-Antikörper geeignet ist für eine Verabreichung innerhalb eines Dosisbereichs von 0,1 µg/kg bis 100 mg/kg.

9. Verwendung nach Anspruch 5, wobei der Antikörper in einem pharmazeutisch verträglichen Träger formuliert ist.

10. Verfahren zum Identifizieren einer Zusammensetzung, welche die Membranfusion zwischen HIV und einer STRL33-positiven Zielzelle oder zwischen einer HIV-infizierten Zelle und einer STRL33-positiven nicht infizierten Zelle blockiert, umfassend:
a) Inkubieren von Komponenten, umfassend die Zusammensetzung und die Zelle nach Anspruch 1 oder 2, unter Bedingungen, die ausreichend sind, dass die Komponenten wechselwirken können;
b) Kontaktieren der Komponenten von Schritt a) mit HIV oder einer HIV-infizierten Zelle; und
c) Messen des Vermögens der Zusammensetzung die Membranfusion zwischen HIV und der STRL33-positiven Zelle oder zwischen einer HIV-infizierten Zelle und einer STRL33-positiven nicht infizierten Zelle zu blockieren, wobei die Zellen keine humanen embryonalen Zellen sind.

11. Verfahren nach Anspruch 10, wobei das Messen des Vermögens der Zusammensetzung zur Blockierung der Membranfusion durch Nachweis eines Reportermittels erfolgt.

12. Verfahren nach Anspruch 11, wobei das Reportermittel aus der Gruppe bestehend aus einem Radioisotop, einer Fluoreszenzverbindung, einer Biolumineszenzverbindung, einer Chemilumineszenzverbindung, einem Metallchelator oder einem Enzym ausgewählt ist.

13. Verfahren nach Anspruch 12, wobei das Reportermittel ein lacZ-Gen ist.

14. Transgenes nicht-humanes Tier mit einem Phänotyp, der durch die Expression eines STRL33-Polypeptids und eines CD4-Polypeptids, welche sonst nicht natürlich in dem Tier vorkommen, **gekennzeichnet** ist, wobei der Phänotyp durch ein Transgen verliehen ist, das in somatischen Zellen und Keimzellen des Tiers vorhanden ist, wobei das Transgen eine STRL33-Polypeptid kodierende Nukleinsäuresequenz und eine CD4-Polypeptid kodierende Nukleinsäuresequenz umfasst.

15. Transgenes nicht-humanes Tier nach Anspruch 14, wobei das Tier eine Maus ist.

16. Transgenes nicht-humanes Tier nach Anspruch 14, wobei das Tier ein Kaninchen ist.

17. Verfahren zur Erzeugung eines transgenen nicht-humanen Tiers mit einem Phänotyp, der durch die Expression eines STRL33-Polypeptids und eines CD4-Polypeptids, welche sonst nicht natürlich in dem Tier vorkommen, **gekennzeichnet** ist, wobei das Verfahren umfasst:
a) Einführen wenigstens eines Transgens in eine Zygote eines Tiers, wobei das Transgen (die Transgene) ein STRL33 kodierendes DNA-Konstrukt und ein CD4 kodierendes DNA-Konstrukt umfasst (umfassen),
b) Transplantieren der Zygote in ein pseudoschwangeres Tier,
c) Entwickelnlassen der Zygote und
d) Identifizieren wenigstens eines transgenen Nachkommens, der das Transgen enthält.

18. Verfahren nach Anspruch 17, wobei das Einführen des Transgens in den Embryo durch Einführen einer embryonalen Stammzelle, welches das Transgen enthält, in den Embryo erfolgt.

19. Verfahren nach Anspruch 17, wobei das Einführen des Transgens in den Embryo durch Infizieren des Embryos mit einem Retrovirus, welches das Transgen enthält, erfolgt.

20. Verfahren nach Anspruch 17, wobei das Tier aus der Gruppe bestehend aus einer Maus und einem Kaninchen ausgewählt ist.

21. Transgenes nicht-humanes Tier, das ein Transgen aufweist, das die Expression von chromosomal in die Keimzellen des Tiers integriertem STRL33 disruptiert oder mit selbiger interferiert.

22. Transgenes Tier nach Anspruch 21, wobei das Tier aus der Gruppe bestehend aus einer Maus und einem Kaninchen ausgewählt ist.

23. Transgenes nicht-humanes Tier nach Anspruch 22, wobei das Transgen ein spezifisches STRL33-Antisense-Polynukleotid umfasst.

24. Verwendung eines spezifischen Anti-STRL33-Antikörpers oder eines Epitop-Bindungsfragments davon oder eine spezifische STRL33-Antisense-Nukleinsäure zur Herstellung eines Arzneimittels für die Behandlung eines Subjekts, das eine HIV-Infektion oder eine HIV-verwandte Erkrankung aufweist oder gefährdet ist, eine HIV-Infektion oder eine HIV-verwandte Erkrankung aufzuweisen, wobei der spezifische Anti-STRL33-Antikörper oder ein Epitop-Bindungsfragment davon oder die spezifische STRL33-Antisense-Nukleinsäure die Zell-Zell-Fusion in mit HIV infizierten STRL33-positiven Zellen inhibiert und wobei die Zellen keine humanen embryonalen Zellen sind.

25. Verwendung nach Anspruch 24, wobei der Antikörper ein monoklonaler Antikörper ist.

26. Verwendung nach Anspruch 25, wobei der monoklonale Antikörper ein humanisierter monoklonaler Antikörper ist.

27. Verwendung nach Anspruch 25, wobei der monoklonale Antikörper geeignet ist für die Verabreichung an einen Patienten, der am erworbenen Immundefektsyndrom (AIDS) oder am Lymphadenopathie-Syndrom bei AIDS (ARC) leidet.

28. Verwendung nach Anspruch 25, wobei der monoklonale Antikörper geeignet ist für die Verabreichung innerhalb eines Dosisbereichs zwischen etwa 0,1 µg/kg bis etwa 100 mg/kg.

29. Verwendung nach Anspruch 25, wobei der monoklonale Antikörper in einem pharmazeutisch verträglichen Träger formuliert ist.

30. Verwendung nach Anspruch 29, wobei der Träger ein Vektor ist.

31. Verwendung nach Anspruch 24, wobei das Arzneimittel geeignet ist zur Verabreichung ex vivo.

32. In vitro-Verfahren zum Nachweisen der Suszeptibilität einer Zelle gegenüber einer HIV-Infektion, umfassend:
- Inkubieren einer ersten Zelle in vitro mit einer zweiten Zelle, welche HIV-env exprimiert, unter Bedingungen, welche eine Fusion der zwei Zellen ermöglichen; und
- Nachweisen der Fusion der Zellen, wobei die Fusion auf das Vorhandensein von STRL33 auf der ersten Zelle und auf die Suszeptibilität gegenüber einer HIV-Infektion hinweist und wobei die Zellen keine humanen embryonalen Zellen sind.

33. Verfahren nach Anspruch 32, wobei die erste oder zweite Zelle ferner ein Reportermittel zum Nachweis der Zellfusion umfasst.

34. Verfahren nach Anspruch 32, wobei die erste Zelle eine T-Zelle ist.

35. Verfahren nach Anspruch 33, wobei das Reportermittel aus der Gruppe bestehend aus einem Radioisotop, einer Fluoreszenzverbindung, einer Biolumineszenzverbindung, einer Chemilumineszenzverbindung, einem Metallchelator oder einem Enzym ausgewählt ist.

36. Verfahren nach Anspruch 35, wobei das Reportermittel ein lacZ-Gen ist.

37. In vitro-Verfahren zum Bestimmen der Suszeptibilität einer Zelle gegenüber einer HIV-Infektion, umfassend das Nachweisen des Vorhandenseins von STRL33 auf der Zelle, wobei das Vorhandensein von STRL33 auf eine Suszeptibilität gegenüber einer HIV-Infektion hinweist und wobei die Zelle keine humane embryonale Zelle ist.

## Revendications

1. Cellule hôte recombinante transformée de manière stable avec un polynucléotide codant pour un polypeptide STRL33, dans laquelle la cellule co-exprime un polypeptide STRL33 et CD4, et dans laquelle la cellule n'est pas une cellule embryonnaire humaine.

2. Cellule hôte recombinante transformée de manière stable avec un polynucléotide codant pour un polypeptide STRL33 et un polynucléotide codant pour un polypeptide CD4, dans laquelle la cellule co-exprime un polypeptide STRL33 et CD4, et dans laquelle la cellule n'est pas une cellule embryonnaire humaine.

3. Cellule selon l'une quelconque des revendications 1 et 2, dans laquelle la cellule est une cellule humaine.

4. Cellule selon l'une quelconque des revendications 1 et 2, dans laquelle la cellule est une cellule non humaine.

5. Utilisation d'un anticorps spécifique anti-STRL33 ou d'un fragment de liaison à un épitope de celui-ci dans la production d'un médicament destiné à inhiber une fusion membranaire entre le virus de l'immunodéficience humaine (VIH) et une cellule cible STRL33 positive ou entre une cellule infectée par le VIH et une cellule non infectée CD4 positive STRL33 positive dans laquelle l'anticorps spécifique anti-STRL33 ou le fragment de liaison à un épitope de celui-ci inhibe une fusion membranaire entre le VIH et une cellule cible STRL33 positive ou bien entre une cellule infectée par le VIH et une cellule non infectée CD4 positive STRL33 positive, et dans laquelle la cellule n'est pas une cellule embryonnaire humaine.

6. Utilisation selon la revendication 5, dans laquelle l'anticorps est un anticorps monoclonal ou un anticorps polyclonal.

7. Utilisation selon la revendication 5, dans laquelle l'anticorps spécifique anti-STRL33 est convenable pour une administration par injections intraveineuses, intramusculaires ou sous-cutanées.

8. Utilisation selon la revendication 5, dans laquelle l'anticorps spécifique anti-STRL33 est convenable pour une administration dans une plage de doses de 0,1 µg/kg à 100 mg/kg.

9. Utilisation selon la revendication 5, dans laquelle l'anticorps est formulé dans un support pharmaceutiquement acceptable.

10. Procédé pour identifier une composition qui bloque la fusion membranaire entre le VIH et une cellule cible STRL33 positive ou bien entre une cellule infectée par le VIH et une cellule non infectée STRL33 positive comprenant :
a) l'incubation de composants comprenant la composition et la cellule selon la revendication 1 ou 2 dans des conditions suffisantes pour permettre aux composants d'interagir ;
b) la mise en contact des composants de l'étape a) avec le VIH ou une cellule infectée par le VIH ; et
c) la mesure de la capacité de la composition à bloquer la fusion membranaire entre le VIH et la cellule STRL33 positive ou bien entre une cellule infectée par le VIH et une cellule non infectée STRL33 positive, dans lequel les cellules ne sont pas des cellules embryonnaires humaines.

11. Procédé selon la revendication 10, dans lequel la mesure de la capacité de la composition à bloquer une fusion membranaire est par détection d'un moyen rapporteur.

12. Procédé selon la revendication 11, dans lequel le moyen rapporteur est choisi dans le groupe constitué d'un radio-isotope, d'un composé fluorescent, d'un composé bioluminescent, d'un composé chimioluminescent, d'un chélateur métallique ou d'une enzyme.

13. Procédé selon la revendication 12, dans lequel le moyen rapporteur est un gène lacZ.

14. Animal transgénique non humain ayant un phénotype **caractérisé par** l'expression d'un polypeptide STRL33 et d'un polypeptide CD4 n'existant pas naturellement autrement dans l'animal, le phénotype étant conféré par un transgène contenu dans les cellules somatiques et germinales de l'animal, le transgène comprenant une séquence d'acide nucléique qui code pour un polypeptide STRL33 et une séquence d'acide nucléique qui code pour un polypeptide CD4.

15. Animal transgénique non humain selon la revendication 14, dans lequel l'animal est une souris.

16. Animal transgénique non humain selon la revendication 14, dans lequel l'animal est un lapin.

17. Procédé pour produire un animal transgénique non humain ayant un phénotype **caractérisé par** l'expression d'un polypeptide STRL33 et d'un polypeptide CD4 n'existant pas naturellement autrement dans l'animal, le procédé comprenant :
(a) l'introduction d'au moins un transgène dans un zygote d'un animal, le(les) transgène(s) comprenant une construction d'ADN codant pour STRL33 et une construction d'ADN codant pour CD4,
(b) la transplantation du zygote dans un animal pseudogravide,
(c) l'action de laisser le zygote se développer jusqu'au terme, et
(d) l'identification d'au moins un descendant transgénique contenant le transgène.

18. Procédé selon la revendication 17, dans lequel l'introduction du transgène dans l'embryon s'effectue par introduction d'une cellule souche embryonnaire contenant le transgène dans l'embryon.

19. Procédé selon la revendication 17, dans lequel l'introduction du transgène dans l'embryon s'effectue par infection de l'embryon avec un rétrovirus contenant le transgène.

20. Procédé selon la revendication 17, dans lequel l'animal est choisi dans le groupe constitué d'une souris et d'un lapin.

21. Animal transgénique non humain ayant un transgène interrompant ou interférant avec l'expression de STRL33 intégré de manière chromosomique dans les cellules germinales de l'animal.

22. Animal transgénique selon la revendication 21, dans lequel l'animal est choisi dans le groupe constitué d'une souris et d'un lapin.

23. Animal transgénique non humain selon la revendication 22, dans lequel le transgène comprend un polynucléotide antisens spécifique de STRL33.

24. Utilisation d'un anticorps spécifique anti-STRL33 ou d'un fragment de liaison à un épitope de celui-ci, ou bien d'un acide nucléique antisens spécifique de STRL33 dans la production d'un médicament destiné à traiter un sujet présentant ou à risque de présenter une infection par le VIH ou un trouble lié au VIH, dans laquelle l'anticorps spécifique anti-STRL33 ou le fragment de liaison à un épitope de celui-ci, ou bien l'acide nucléique antisens spécifique de STRL33 inhibe une fusion cellule-cellule dans des cellules STRL33 positives infectées par le VIH, et dans laquelle les cellules ne sont pas des cellules embryonnaires humaines.

25. Utilisation selon la revendication 24, dans laquelle l'anticorps est un anticorps monoclonal.

26. Utilisation selon la revendication 25, dans laquelle l'anticorps monoclonal est un anticorps monoclonal humanisé.

27. Utilisation selon la revendication 25, dans laquelle l'anticorps monoclonal est convenable pour une administration à un patient souffrant d'un Syndrome d'Immuno-déficience Acquise (SIDA) ou d'un complexe lié au SIDA (ARC).

28. Utilisation selon la revendication 25, dans laquelle l'anticorps monoclonal est convenable pour une administration dans une plage de doses entre environ 0,1 µg/kg jusqu'à environ 100 mg/kg.

29. Utilisation selon la revendication 25, dans laquelle l'anticorps monoclonal est formulé dans un support pharmaceutiquement acceptable.

30. Utilisation selon la revendication 29, dans laquelle le support est un vecteur.

31. Utilisation selon la revendication 24, dans laquelle le médicament est convenable pour une administration ex vivo.

32. Procédé in vitro pour détecter la sensibilité d'une cellule à une infection par le VIH comprenant :
l'incubation d'une première cellule in vitro avec une deuxième cellule qui exprime HIV-env dans des conditions qui permettent une fusion des deux cellules ; et
la détection d'une fusion des cellules, dans laquelle une fusion est indicatrice de la présence de STRL33 sur la première cellule et la sensibilité à une infection par le VIH, et dans lequel les cellules ne sont pas des cellules embryonnaires humaines.

33. Procédé selon la revendication 32, dans lequel la première ou la deuxième cellule comprend en outre un moyen rapporteur pour la détection d'une fusion cellulaire.

34. Procédé selon la revendication 32, dans lequel la première cellule est une cellule T.

35. Procédé selon la revendication 33, dans lequel le moyen rapporteur est choisi dans le groupe constitué d'un radio-isotope, d'un composé fluorescent, d'un composé bioluminescent, d'un composé chimioluminescent, d'un chélateur métallique ou d'une enzyme.

36. Procédé selon la revendication 35, dans lequel le moyen rapporteur est un gène lacZ.

37. Procédé in vitro pour déterminer la sensibilité d'une cellule à une infection par le VIH comprenant la détection de la présence de STRL33 sur la cellule, dans lequel la présence de STRL33 est indicatrice d'une sensibilité à une infection par le VIH, et dans lequel la cellule n'est pas une cellule embryonnaire humaine.
